# EUROPEAN PATENT SPECIFICATION

(11) **EP 3 295 952 B1**
(45) Date of publication and mention of the grant of the patent: **17.02.2021**
(21) Application number: 16792203.8
(22) Date of filing: 12.05.2016
(51) Int. Cl.: A61K 38/26, A61K 9/08, A61K 47/10, A61P 3/10, A61K 47/26

(54) **PHARMACEUTICAL FORMULATION COMPRISING GLP-1 ANALOGUE AND PREPARATION METHOD THEREOF**
PHARMAZEUTISCHE FORMULIERUNG MIT GLP-1-ANALOG UND HERSTELLUNGSVERFAHREN DAFÜR
FORMULATION PHARMACEUTIQUE COMPRENANT UN ANALOGUE DU GLP-1 ET SON PROCÉDÉ DE PRÉPARATION

(30) Priority: 13.05.2015 CN 201510243040
(43) Date of publication of application: 21.03.2018
(73) Proprietor: Hangzhou Jiuyuan Gene Engineering Co., Ltd, Hangzhou, Zhejiang 310018 (CN)
(72) Inventor: LIU, Xiaoni, Hangzhou Zhejiang 310018 (CN); LI, Hanxing, Hangzhou Zhejiang 310018 (CN); CHEN, Chaoliang, Hangzhou Zhejiang 310018 (CN); MA, Guochang, Hangzhou Zhejiang 310018 (CN); XU, Feihu, Hangzhou Zhejiang 310018 (CN); WANG, Tongying, Hangzhou Zhejiang 310018 (CN); SUN, Handong, Hangzhou Zhejiang 310018 (CN)
(74) Representative: ABG Intellectual Property Law, S.L.
(86) International application number: PCT/CN2016/081843
(87) International publication number: WO 2016/180353

(56) References cited:
- WO-A1-2014/030051
- CA-A1- 2 921 250
- CN-A- 1 195 992
- CN-A- 1 882 356
- CN-A- 102 085 355
- CN-A- 102 085 355
- CN-A- 103 405 753
- CN-A- 103 893 744
- CN-A- 103 893 744
- CN-A- 104 548 096
- US-A1- 2008 125 361
- US-A1- 2009 286 716
- US-A1- 2010 234 299
- US-A1- 2012 225 810

## Description

### FIELD OF THE INVENTION

The present invention relates to a pharmaceutical formulation comprising peptide drug. More particularly, the present invention relates to a pharmaceutical preparation comprising GLP-1 analog and a process for the preparation thereof.

### BACKGROUND OF THE INVENTION

In the 1960s, McIntyre and Elrick found that the effect of oral glucose on insulin secretion was significantly higher than that of intravenous injection, and this additional effect was called "incretin effect". With the development of cell and molecular biology, studies have confirmed that Incretin is a kind of intestinal hormone in the human body. After eating, the hormone can promote insulin secretion and exert glucose-dependent hypoglycemic effect.

Incretin is mainly composed of Glucagon-like peptide-1 (GLP-1) and sugar-dependent insulin-releasing peptide (GIP), in which GLP-1 plays a more important role in the development and progression of type 2 diabetes mellitus. Studies have shown that GLP-1 can reduce blood glucose by stimulating insulin secretion, inhibiting glucagon secretion and gastric emptying. On the other hand, GLP-1 also has a unique role in slowing beta cell apoptosis and promoting its regeneration.

In 1983, McIntyre et al. found GLP-1 in the analysis of the gene sequence of glucagon precursor (proglucagon, PG). GLP-1 gene is expressed in pancreatic α-cells and intestinal L cells. The complete GLP-1 polypeptide consists of 37 amino acids whose 1-37 peptide sequence is:
HDEFERHAEGTFTSDVSSYLEGQAAKEFIAWLVKGRG;

The bioactive forms of of GLP-1 in vivo is GLP-1 (7-37) peptide and GLP-1 (7-36) amide in which about 80% GLP-1 cyclic activity is derived from GLP-1 (7-36) amide. The amino terminus of GLP-1 (7-37)-OH is designated No.7 and the carboxy terminus is designated No. 37, as is customary in the art. For a more detailed description of GLP-1 analogs and derivatives, see Hoffmann, JA [WO99/ 29336, published June 17, 1999] and Knudsen, LB et al. [J. Med. Chem. 43: 1664-1669 (2000)]. After GLP-1 (1-37) was formed in vivo, the N-terminal 6 amino acids were removed and the C-terminal amide was formed by two-step Enzyme digestion to finally generate the highly active GLP-1 (7-36) amide (also known as GLP-1 fragment).

GLP-1 is the strongest intestinal peptide hormone that has been found to promote insulin secretion, which acts by binding to the GLP-1 receptor (GLP-1R, a G-coupled protein belonging to the β-receptor family). GLP-1 binds to GLP-1R, activates intracellular cyclic adenosine monophosphate (cAMP) and mitogen-activated protein kinase (MAPK) pathway. Islet mature β cells of the GLP-1 receptor couples Gs, activated adenylyl cyclase, increases cAMP production, the latter and glucose synergistically stimulate insulin synthesis and secretion, stimulate insulin gene transcription and insulin biosynthesis, reduce the concentration of glucagon and inhibit glucagon secretion, enhance cell sensitivity to insulin, stimulate insulin-dependent glycogen synthesis, reduce postprandial blood glucose levels. By activating protein kinase, phosphatidylinositol 3-kinase (PI3K) and MAPK pathway, GLP-1 can regulate pre -apoptotic proteins and induce the expression of anti-apoptotic protein Bcl-2 and Bcl-xL to slow down β-cell apoptosis, enhance its regeneration, promote islet β-cell differentiation and proliferation. In addition, GLP-1 can slow down gastric emptying speed, suppress appetite by acting on the hypothalamus.

At present, GLP-1 and its analogs are prepared by gene recombination. Gene recombinant preparation of protein, peptides often uses E. coli and yeast as a host. Among them, liraglutide has been approved and occupy the mainstream of GLP-1 analog drug market,trade name is Victoza. Liraglutide's chemical name is Arg³⁴, Lys²⁶ (N^{ε}- (γ-Glu (N^{α}-hexadecanoyl))) GLP-1 (7-37), a fatty acid-modified GLP-1 analog, its plasma half-life is extended to 13 hours after subcutaneous administration. Liraglutide's GLP-1 sequence is similar to human GLP-1 (7-37) sequence, in which the 34th lysine is replaced by arginine, and its amino acid sequence has about 97% homology with human native GLP-1 (7-37) (Goke et al., J. Biol. Chem., 268: 19650-55, 1993). A 16 carbon fatty acid is linked to lysine at GLP-1 position 26 through a glutamic acid and its structure is as follows:

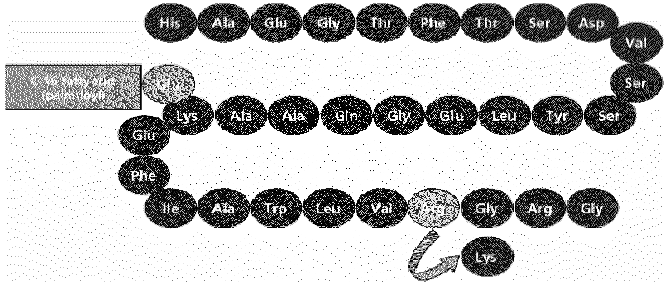
C-16 fatty acid
(palmitoyl)
His Ala Glu Gly Thr Phe Thr Ser Asp
Val
Ser
Glu
Lys Ala Ala Gln Gly Glu Leu Tyr Ser
Glu
Phe
Ile Ala Trp Leu Val Arg Gly Arg Gly
Lys

As a GLP-1 analog, liraglutide has the following pharmacological effects due to high homology with GLP-1: (1) enhance insulin secretion by blood glucose concentration-dependent manner; (2) inhibit postprandial glucagon secretion; (3) suppress appetite, slow down gastric emptying. In addition, liraglutide can promote βcell proliferation and differentiation, regulate β cell apoptosis gene-related expression and inhibit apoptosis, increase the number of islet βcells in vivo. Studies have shown that liraglutide can also restore the sensitivity of human isletβcells to blood glucose.

Usually the first-line treatment of type 2 diabetes is oral hypoglycemic drugs, but due to the oral hypoglycemic drugs' adverse reactions, especially hypoglycemia, so that it does not apply to all patients with type 2 diabetes. Studies have shown that liraglutide has a good therapeutic outlook for diabetes. LEAD (Liraglutide Effect and Action in Diabetes) studies in more than 5,000 diabetic patients in more than 40 countries have shown that liraglutide has the effect of protecting the function of isletβcells and improving the amount and quality of βcell insulin secretion , so it is possible to fundamentally change the course of diabetes development. The LEAD study included six phase III clinical trials that evaluated extensively the efficacy of liraglutide monotherapy and combined with other oral hypoglycemic drugs. Studies have shown that liraglutide can not only effectively protect the function of isletβcells, delay the progress of type 2 diabetes, but also be able to quickly, efficiently and permanently reduce glycosylated hemoglobin (HbA1c), and rarely occurs hypoglycemia, as well as lose body weight and reduce the role of systolic blood pressure.

Clinical trials have shown that liraglutide has a protective effect on the cardiovascular system. The study found that liraglutide can reduce systolic blood pressure by 2.7 ∼ 6.7 mmHg, this phenomenon occurs before the decline in weight of patients, so the decline in systolic blood pressure can not be completely attributed to changes in patient weight. The study found that for patients receiving liraglutide treatment pulse rate increased slightly, the clinical significance of this phenomenon remains to be further studied. Courreges et al. reported that liraglutide treatment was accompanied by a decrease in total cholesterol, low density lipoprotein, free fatty acid, triglyceride, plasminogen activator inhibitor-1 and B-type natriuretic peptide concentrations. The study also found that the levels of high-sensitivity C-reactive protein in patients with liraglutide treatment showed a dose-dependent decrease, although not significant.

As a GLP-1 analog,liraglutide's glucose-lowering effect is glucose-dependent, which promotes insulin secretion and inhibits Glucagon action only when the blood glucose concentration is high. Therefore, the use of liraglutide alone produces almost no hypoglycemia. Clinical trials have shown that the incidence of hypoglycemia in liraglutide monotherapy is significantly lower than that of glimepiride, comparable to metformin. liraglutide combination therapy causes hypoglycemia events that are comparable or less than other hypoglycemic drugs. Nauck et al reported that the incidence of hypoglycemia in patients treated with liraglutide combined with metformin was comparable to that of placebo plus metformin, which was less than 3%. The incidence of hypoglycemia was 17% in patients treated with glimepiride combined with metformin, the difference was statistically significant (P <0.001).

The most common adverse reactions in liraglutide treatment are gastrointestinal reactions, mainly manifestations of nausea, vomiting, and diarrhea, which are usually seen in a dose-dependent manner in the first week of treatment. In LEAD-2 and LEAD-3 clinical trials, patients with nausea in the liraglutide treatment group were <10%. By slowly increasing the dose can reduce the incidence of gastrointestinal reactions, patients often in the medication 1 to 3 weeks to tolerate this adverse reaction, few patients discontinue treatment because of gastrointestinal reactions.

As a new generation of hypoglycemic drug, liraglutide alone or in combination with other oral hypoglycemic drugs can quickly and efficiently reduce blood glucose and HbA1c levels. Its hypoglycemic effect depends on the glucose concentration, that is, only with increased the blood glucose level to stimulate the release of insulin to achieve therapeutic effect, so the probability of hypoglycemia is very low. The most noteworthy is its potential to delay the progress of diabetes. A large number of clinical trials have shown that liraglutide can improve βcell function, reduce its apoptosis, increase βcell newborn, and thus delay the progress of diabetes. The protective effect of liraglutide on the cardiovascular system allows it to reduce the incidence of cardiovascular complications in diabetes. In addition, liraglutide has a significant effect on weight loss and is suitable for patients who need to lose weight and patients with severe hypoglycemia risk. The unique pharmacological effects of liraglutide have a broad prospect in the treatment of diabetes.

The production process of liraglutide had been reported, see Chinese Patent 97198413.1 and 99808706.8. It should be noted that, GLP-1 protein is a class of more easily degradable protein, a variety of hydrolytic enzymes easy to promote its degradation (M. Egel-Mitani, et al .; Yield improvement of heterologous peptides expressed in yps1-disrupted Saccharomyces cerevisia lines ; Enzyme and Microbial Technology, 2000, 26: 671-677). Although liraglutide is more stable through fatty acid chain modification, as a kind of peptide drug, its stability can not be compared with conventional chemical drug. Its physical and chemical, biological properties in the long-term storage will be affected by a variety of environmental factors . For example, liraglutide is highly sensitive to temperature, oxygen and ultraviolet light. Due to these factors, a variety of physical or chemical changes, such as adsorption, polymerization, precipitation and oxidation, may occur due to the effects of these factors, which makes it extremely difficult to choose the formulation, ensuring 90% of the purity in the drug shelf life. This standard is much lower than the standard of the usual protein drug preparation in 95% purity. Therefore, if the stability of liraglutide during storage can not be guaranteed, it will lead to changes in dose and thus affect the efficacy.

Visible foreign matter in the formulation solution is usually caused by the physical oscillations, the interaction of protein molecules in the solution and the interaction of protein molecules at the liquid-gas interface in the vial. It is believed that protein molecules are adsorbed on the gas-liquid interface, and their hydrophobic groups extend into the air while the hydrophilic groups are immersed in the aqueous phase. Once arranged on the surface, the protein molecules are easily gathered to form particles and precipitate. It is also believed that further conformational changes in the proteins adsorbed at the gas-liquid and solid-liquid interfaces due to vibration during transportation or other processes. This oscillation can cause protein entanglement, aggregation, forming particles and eventually precipitation with other adsorbed proteins. In addition, the protein in the freeze-drying process due to pre-freeze rate, heating rate and other freeze-drying curve of the process results in visible foreign matter.

Chinese patent application CN200480034152.8 refers to an improved formulation of liraglutide. In the past experiments it has been found that the liraglutide is easily crystallized from the pharmaceutical solution, for example, the formulation containing mannitol causes clogging of the production device or the needle of the syringe due to its crystallization, thereby affecting the production control of the device or the treatment of the patient. After a lot of exploration experiments, mannitol, glycerol, sucrose, PEG400, arginine, dimethylsulfone, sorbitol, inositol, glucose, glycine, maltose and lactose were excluded, the propylene glycol was ultimately chosen as an isotonic regulator of liraglutide formulation and it was found that formulations containing propylene glycol had no effect on the physical and chemical stability of liraglutide (but no data was provided) and the preparation is not easy to produce sediment.

However, since liraglutide is used for the treatment of diabetes, it is necessary to administer it for life and inject it every day. Liraglutide prescription was listed as: 3ml solution containing 18mg of liraglutide, 1.42mg disodium hydrogen phosphate dihydrate, 14mg propylene glycol, 5.5mg phenol, dissolved in water for injection, pH 8.15. Although propylene glycol is relatively safe to use as an adjuvant materials, but according to clinical summary, propylene glycol has some possible side effects on human skin such as:
(1) irritation: some people will have the subjective sense of burning, tingling and itching during use.
(2) Defatting: propylene glycol with a fat-soluble solvent properties, long-term use of high concentrations of propylene glycol will have an impact on the skin sebum structure.
(3) irritant dermatitis: propylene glycol will irritate the skin and mucous membranes, the higher the concentration and the more sealed use situation, the greater the irritation, and will cause skin redness, rash, peeling itching and rough situation. Although for the majority of people it is not much stimulating, but the long-term use may have the cumulative effect, it is worth noting. For the injured skin or sensitive skin, even low concentrations of propylene glycol is easy to produce stimulating response.
(4) allergic dermatitis: about 1 to 5% of people exposed to propylene glycol will produce local skin allergic eczema reaction. Usually the first contact will not be a problem, but once again exposed, it will produce skin allergies.
(5) systemic contact dermatitis: A small number of people who produce skin allergies for propylene glycol, if taken or applied with food or drugs containing propylene glycol ingredients, will cause systemic skin allergies.

In general, the use of propylene glycol is quite extensive and the safety is fairly high, but there is potentially irritation and sensitization to the skin. For its common use, if there are other safer alternative ingredients, it is possible to minimize the use of propylene glycol or reduce its use of the concentration, the skin will be more secure.

Therefore, it has become extremely important to develop a pharmaceutical preparation which can replace propylene glycol as a pharmaceutical excipient of liraglutide formulation, so that promote the liraglutide not easy to be degraded and prevents crystallization from the preparation solution and is suitable for practical clinical use. The present invention relates to this aspect.

### DESCRIPTION OF THE INVENTION

This invention has unexpectedly obtained a stable pharmaceutical composition comprising a GLP-1 analog by lots of prescription screening and stability tests.

The present invention relates to a pharmaceutical formulation comprising GLP-1 analog in a concentration from 0.1mg/ml to 25mg/ml, a buffer with pH from 7.5 to 9.0, a stabilizer in a concentration from 0.001% to 0.5% (m/v), xylitol in a concentration from 0.5% to 10%(m/v), and preservative in a concentration from 0.1mg/ml to 10 mg/ml
wherein the GLP-1 analog is selected from the group consisting of GLP-1 or its mutant, GLP-1(7-36)-amide or its mutant, GLP-1 (7-37) or its mutant, and GLP-1 derivatives which is chemically modified by introducing an organic substituent e.g. ester, alkyl or lipophilic group on one or more amino acid residues of the above peptide or its mutant thereof;
and wherein the term "mutant" designates the peptide GLP-1, GLP-1 (7-36)-amide or GLP-1 (7-37) wherein one or more amino acid residues of the peptide have been substituted by another amino acid residue and/or wherein one or more amino acid residues of the peptide have been deleted and/or wherein one or more amino acid residues have been added to the peptide, and wherein the mutant is a peptide GLP-1, GLP-1 (7-36) -amide or GLP-1 (7-37) wherein 6 or less amino acids have been substituted and/or added and/or deleted.

In the present invention, the term "GLP-1 analog" is understood to refer to GLP-1 or its mutant, GLP-1(7-36)-amide or its mutant, GLP-1 (7-37) or its mutant, GLP-1 derivatives which is chemically modified by introducing an organic substituent e.g. ester, alkyl or lipophilic group on one or more amino acid residues of the above peptide or its mutant thereof. The term "mutant" is used to designate the parent peptide GLP-1, GLP-1 (7-36) -amide or GLP-1 (7-37) wherein one or more amino acid residues of the parent peptide have been substituted by another amino acid residue and/or wherein one or more amino acid residues of the parent peptide have been deleted and/or wherein one or more amino acid residues have been added to the parent peptide. Such addition can take place either at the N-terminal end or at the C-terminal end of the parent peptide or both. Preferably, the mutant is a peptide wherein 6 or less amino acids have been substituted and/or added and/or deleted from the parent peptide, more preferably a peptide wherein 3 or less amino acids have been substituted and/or added and/or deleted from the parent peptide, and most preferably, a peptide wherein one amino acid has been substituted and/or added and/or deleted from the parent peptide.

In one embodiment of the invention, the GLP-1 derivative preferably has three lipophilic substituents, more preferably two lipophilic substituents, and most preferably one lipophilic substituent attached to the parent peptide (ie GLP-1(7-36)-amide, GLP-1(7-37), a GLP-1(7-36)-amide analog or a GLP-1(7-37) analog), where each lipophilic substituent(s) preferably has 4-40 carbon atoms, more preferably 8-30 carbon atoms, even more preferably 8-25 carbon atoms, even more preferably 12-25 carbon atoms, and most preferably 14-18 carbon atoms.

GLP-1 analogs suitable for the present invention are described in the prior art includes those referred to WO93/19175 (Novo Nordisk), WO99/ 43705 (Novo Nordisk), WO99/43706 (Novo Nordisk), WO99/43707 (Novo Nordisk), WO98/08871 (analogs with lipophilic substituents) and in WO02/ 46227 (analogs fused to serum albumin or to Fc portion of an Ig) (Novo Nordisk A/S), WO99/43708 (Novo Nordisk A/S), WO99/43341 (Novo Nordisk A/S), WO87/06941 (The General Hospital Corporation), WO90/ 11296(The General Hospital Corporation), WO91/11457(Buckley et al.), WO98/43658(Eli Lilly & Co.), EP0708179-A2(Eli Lilly & Co.), EP0699686-A2(Eli Lilly & Co.), WO01/98331(Eli Lilly & Co.) And those mentioned in CN200480034152.8, Which is hereby incorporated by reference in its entirety.

In one embodiment of the present invention, the GLP-1 analog is preferably Arg³⁴,Lys²⁶(N^{ε}-(γ-Glu(N^{α}-hexadecanoyl)))-GLP-1(7-37), refers to Liraglutide.

In yet another embodiment of the present invention, the GLP-1 analog is selected from the group consisting of : Gly⁸-GLP-1(7-36)-amide, Gly⁸-GLP-1(7-37), Val⁸-GLP-1(7-36)-amide, Val⁸-GLP-1(7-37), Val⁸Asp²²-GLP-1(7-36)-amide, Val⁸Asp²²-GLP-1(7-37), Val⁸Glu²²-GLP-1 (7-36)-amide, Val⁸Glu²²-GLP-1(7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1 (7-37), Val⁸His²²-GLP-1(7-36)-amide, Val⁸His²²-GLP-1(7-37), analogs thereof and derivatives of any of these.

In yet another embodiment of the present invention, the GLP-1 analog is selected from the group consisting of : Arg²⁶-GLP-1(7-37); Arg³⁴-GLP-1(7-37); Lys³⁶-GLP-1(7-37); Arg^{26, 34}Lys³⁶-GLP-1(7-37); Arg^{26, 34}-GLP-1(7-37); Arg^{26, 34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly⁸His²²-GLP-1(7-37); Val⁸His²²-GLP-1(7-37); Met⁸His²²-GLP-1 (7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Val⁸Asp²²-GLP-1(7-37); Met⁸Asp²²-GLP-1(7-37); Gly⁸Glu²²-GLP-1(7-37); Val⁸Glu²²-GLP-1(7-37); Met8Glu²²-GLP-1(7-37); Gly⁸Lys²²-GLP-1(7-37); Val⁸Lys²²-GLP-1(7-37); Met⁸Lys²²-GLP-1(7-37); Gly⁸Arg²²-GLP-1(7-37); Val⁸Lys²²His³⁷-GLP-1 (7-37); Gly⁸Glu²²His³⁷-GLP-1(7-37); Val⁸Glu²²His³⁷-GLP-1(7-37); Met⁸Glu²²His³⁷-GLP-1(7-37); Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1(7-37); Val⁸His²²His³⁷-GLP-1(7-37); Met⁸His³³His³⁷-GLP-1(7-37); Gly⁸His³⁷-GLP-1(7-37); Val⁸His³⁷-GLP-1(7-37); Met⁸His³⁷- GLP-1(7-37); Gly⁸Asp²²His³⁷-GLP-1(7-37); Val⁸Asp²²His³⁷-GLP-1 (7-37); Met⁸Asp²²His³⁷-GLP-1(7-37); Arg²⁶-GLP-1(7-36)-amide; Arg³⁴-GLP-1(7-36)-amide; Lys³⁶-GLP-1(7-36)-amide; Arg^{26, 34}Lys³⁶-GLP-1(7-36)-amide; Ar^{26, 34}-GLP-1(7-36)-amide; Arg^{26, 34}Lys⁴⁰-GLP-1(7-36)-amide; Arg²⁶LyS³⁶-GLP-1(7-36)-amide; Arg³⁴Lys³⁶-GLP-1(7-36)-amide; Gly⁸-GLP-1(7-36)-amide; Val⁸-GLP-1(7-36)-amide; Met⁸-GLP-1(7-36)-amide; Gly⁸Asp²²-GLP-1(7-36)-amide; Gly⁸Glu²²His³⁷-GLP-1(7-36)-amide; Val⁸Asp²²-GLP-1(7-3 6)-amide; Met⁸Asp²²-GLP-1(7-3 6)-amide; Gly⁸Glu²²-GLP-1(7-36)-amide; Val⁸Glu²²-GLP-1(7-36)-amide; Met⁸Glu²²-GLP-1(7-3 6)-amide; Gly⁸Lys²²-GLP-1(7-3 6)-amide; Val⁸Lys²²-GLP-1(7-36)-amide; Met⁸Lys²²-GLP-1(7-36)-amide; Gly⁸His²²His³⁷-GLP-1(7-3 6)-amide; Gly⁸Arg²²-GLP-1(7-3 6)-amide; Val⁸Arg²²-GLP-1(7-36)-amide; Met⁸Arg²²-GLP-1(7-36)-amide; Gly⁸His²²-GLP-1(7-3 6)-amide; Val⁸His²²-GLP-1(7-36)-amide; Met⁸His²²-GLP-1(7-36)-amide; His³⁷-GLP-1(7-36)-amide; Val^{g}Arg²²His³⁷-GLP-1(7-3 6)-amide; Met⁸Arg²²His³⁷-GLP-1(7-36)-amide; Gly⁸His³⁷-GLP-1(7-36)-amide; Val⁸His³⁷-GLP-1(7-36)-amide; Met⁸His³⁷-GLP-1(7-36)-amide; Gly⁸Asp²²His³⁷-GLP-1(7-36)-amide; Val⁸Asp²²His³⁷-GLP-1(7-36)-amide; Met⁸Asp²²His³⁷-GLP-1(7-36)-amide; Val⁸Glu²²His³⁷-GLP-1(7-36)-amide; Met⁸Glu²²His³⁷-GLP-1(7-36)-amide; Gly⁸Lys²²His³⁷-GLP-1(7-36)-amide; Val⁸Lys²²His³⁷-GLP-1(7-36)-amide; Met⁸Lys²²His³⁷-GLP-1 (7-3 6)-amide; GlyArg²²His³⁷-GLP-1(7-36)-amide; Val⁸His²²His³⁷-GLP-1(7-36)-amide; Met⁸His²²His³⁷-GLP-1(7-36)-amide;
and derivatives of any of these.

In yet another embodiment of the present invention, the GLP-1 analog is selected from the group consisting of: Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), analogs thereof and derivatives of any of these.

In the pharmaceutical composition disclosed in the present invention, the concentration of the GLP-1 analog is preferably about 1mg/ml to 15mg/ml, more preferably 3mg/ml to 10mg/ml, and most preferably 6mg/ml.

In the pharmaceutical composition disclosed in the present invention, the buffer suitable for use in the present invention is any buffer capable of maintaining the pH of the formulation in the aqueous solution at a pH from 7.5 to 9.0, which can be selected from phosphate buffer, a disodium hydrogen phosphate-Citrate buffer,TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof. The pH of the buffer is preferably in the range of 7.5 to 8.5, more preferably 8.0 to 8.5; the concentration of the buffer is 5 to 100 mmol/L, preferably 10 to 30 mmol/L. Preferably the buffer is the disodium hydrogen phosphate buffer which concentration is in the range of 5 to 100mmol/L and the pH is in the range of 7.5 to 8.5; more preferably the concentration is in the range of 10 to 30 mmol/L and the pH is in the range of 8.0 to 8.5.

In the pharmaceutical composition disclosed in the present invention, xylitol is used as an isotonicity regulator, and the concentration is in the range of 0.5% to 10% (m/v), more preferably in the range of 1% to 5% (m/v).

In the pharmaceutical composition disclosed in the present invention, stabilizers for improving the stability of GLP-1 analogs include, but are not limited to: amino acids and derivatives: glycine, alanine, serine, aspartic acid, glutamic acid, threonine, tryptophan, lysine, hydroxy lysine, histidine, arginine, cystine, cysteine, methionine, phenylalanine, leucine, isoleucine Amino acids and their derivatives; nonionic surfactants: sorbitan fatty acid esters, glycerol fatty acid esters (e.g., sorbitan monoate, sorbitan monolaurate and sorbitan palm Acid monoester), polyglycerol fatty acid esters (e.g., glyceryl octanoic acid monoester, glyceryl myristate mono-tallow cream and glycerol hard fatty acid monoester), polyoxyethylene sorbitan fatty acid esters, polyoxyethylene sorbitol fatty acid esters , Polyoxyethylene glycerol fatty acid esters, polyoxyethylene glycol fatty acid esters, polyoxyethylene alkyl ethers, polyoxyethylene polyoxypropylene alkyl ethers, polyoxyethylene phenyl ethers, polyoxyethylated hard castor oil, Polyoxyethylated beeswax derivatives, polyoxyethylenated lanolin derivatives Or a polyoxyethylene fatty acid amide, the cationic surfactant is an alkyl sulfate (e.g., a C10-C18 alkyl alkyl sulfate); polyethylene glycol, polyvinyl alcohol, hydroxypropyl- Dextrins, carboxymethylcellulose, polyvinylpyrrolidone, polysorbate 20 or polysorbate 80, poloxamer series (poloxamer 124, poloxamer 181, poloxamer 182, poloxamer 188, poloxamer 237, poloxamer 331, poloxamer 338 or poloxamer 407). The stabilizers are preferably polysorbate 20, polysorbate 80, poloxamer 124, poloxamer 181, poloxamer 182, poloxamer 188, poloxamer 237, poloxamer 331, poloxamer 338, poloxamer 407 or a mixture thereof, more preferably polysorbate 20, polysorbate 80, poloxamer 188 or a mixture thereof.

The pharmaceutical composition disclosed herein comprises a pharmaceutically acceptable preservative. Suitable pharmaceutically acceptable preservatives may be selected from the group consisting of phenol, o-cresol, m-cresol, p-cresol, methyl p-hydroxybenzoate, propyl p-hydroxybenzoate, 2-phenoxyethanol, p-hydroxybenzene Butyl formate, 2-phenylethanol, benzyl alcohol, chlorobutanol, chlorocresol, ethyl p-hydroxybenzoate or mixtures thereof at a concentration in the range of about 0.1mg/ml to 10mg/ml, preferably at a concentration in the range of about 1mg/ml to 8mg/ml, and most preferably at a concentration in the range of about 2mg/ml to 6 mg/ml.

The present invention also discloses a preparation method of a GLP-1 analog pharmaceutical composition comprising the steps of:
(1) dissolving a preservative, xylitol and a buffering agent in water for injection to prepare a solution;
(2) dissolving the GLP-1 analogous in the above solution and adjusting to the desired pH range;
(3) adding a stabilizer to the above solution to obtain pharmaceutical formulation comprising GLP-1 analog in a concentration from 0.1mg/ml to 25mg/ml, a buffer with pH from 7.5 to 9.0, a stabilizer in a concentration from 0.001% to 0.5% (m/v), xylitol in a concentration from 0.5% to 10%(m/v), and preservative in a concentration from 0.1mg/ml to 10 mg/ml.

Wherein the definition of preservatives, buffers, stabilizers and GLP-1 analog is as previously described.

After a lot of experiments, the inventors have surprisingly found that the pharmaceutical compositions disclosed herein are particularly suitable for long-term preservation of liraglutide formulation. Liraglutide is administered subcutaneously, and the inventor has found that it is necessary to add a suitable amount of a surfactant to the formulation solution in order to stabilize the liraglutide preparation and to make it not easy to precipitate and crystallize from the solution. However, the type and characteristics of surfactants that are incorporated into the parenteral compositions for injection into the human body are limited in view of the safety of clinical application. Accordingly, there is a need in the art to provide pharmaceutical compositions that can improve protein stability, and in which contain only those commercially viable parenteral ingredients that are considered safe and are included in authoritative approval regulations.

The present invention has surprisingly found that the use of xylitol as an isotonic regulator in place of propylene glycol and the combination of low concentrations of poloxamer surfactants with polysorbate surfactants (preferably Tween-20 or Tween-80) can improve the long-term stability of liraglutide preparation, stored at 4°C for 3 months, the purity of liraglutide is still maintained at 95% or more, compared with Victoza prescription more stable; and the solution keep clear, no visible foreign matter or crystallization which could lead to injection syringe clogging; and xylitol is more safer and less side effects than propylene glycol in the clinical treatment of diabetes

More importantly, the inventors have surprisingly found that xylitol combined with low concentrations of poloxamer surfactant and polysorbate surfactant can reduce the production of high molecular impurities such as dimers during long-term preservation of liraglutide formulation.This unexpected technical effect is never reported in the prior art literature.

Xylitol has a very low glycemic index, is a natural stabilizer for insulin, and its metabolism does not depend on insulin, it does not increase blood glucose levels, and can reduce the abnormal symptoms of diabetic patients such as polydipsia, polyuria and food cravings, so it is a safe diet of diabetes, nutritional supplements and adjuvant therapy. In addition, xylitol has a high degree of chemical stability, which means that it does not interact with drugs and other excipients and can be used over a wide pH range (pH 1-11). Second, xylitol has a lower activity and a higher osmotic pressure in the water, thus increasing the stability of the product. In addition, the literature reported that xylitol can play a synergistic effect on the preservative, while it possesses both bactericidal and bacteriostatic activity, so xylitol can enhance the stability of the product.

The invention also discloses a preparation method of a liraglutide pharmaceutical composition, which comprises the following steps:
(1) dissolving a preservative, xylitol and a buffering agent in water for injection to prepare a solution;
(2) dissolving the liraglutide in the above solution, adjusting the pH to the desired pH;
(3) adding a stabilizer to the above solution to obtain pharmaceutical formulation comprising liraglutide in a concentration from 0.1mg/ml to 25mg/ml, a buffer with pH from 7.5 to 9.0, a stabilizer in a concentration from 0.001% to 0.5% (m/v), xylitol in a concentration from 0.5% to 10%(m/v), and preservative in a concentration from 0.1mg/ml to 10 mg/ml.

The pharmaceutical formulation prepared by the present invention comprising liraglutide in a concentration from 0.1mg/ml to 25mg/ml, a buffer has pH from 7.5 to 9.0, a stabilizer in a concentration from 0.001% to 0.5% (m/v), xylitol in a concentration from 0.5% to 10%(m/v), and preservative in a concentration from 0.1mg/ml to 10 mg/ml.The present invention has the advantage of providing a formulation suitable for clinical multiple injections by adding some ingredients which can be accepted by the human body and enhancing the stabilization of the physicochemical and biological activity of liraglutide. This preparation can effectively prevent the active ingredient liraglutide to form high molecular polymer, reduced purity or crystallization due to degradation, oxidation, precipitation and other factors caused, so as to facilitate the transport, long-term preservation and clinical use.

In the pharmaceutical composition disclosed in the present invention, the concentration of the liraglutide is preferably about 1mg/ml to 15mg/ml, more preferably 3mg/ml to 10mg/ml, and most preferably 6mg/ml.

in the pharmaceutical composition disclosed in the present invention, the buffer suitable for use in the present invention is any buffer capable of maintaining the pH of the formulation in the aqueous solution at a pH of 7.5 to 9.0, which can be selected from phosphate buffer, a disodium hydrogen phosphate-Citrate buffer,TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof. The pH of the buffer is preferably in the range of 7.5 to 8.5, more preferably 8.0 to 8.5; the concentration of the buffer is 5 to 100 mmol/L, preferably 10 to 30 mmol/L. Preferably the buffer is the disodium hydrogen phosphate buffer which concentration is in the range of 5 to 100mmol/L and the pH is in the range of 7.5 to 8.5; more preferably the concentration is in the range of 10 to 30 mmol/L and the pH is in the range of 8.0 to 8.5.

The said pharmaceutical composition uses xylitol as an isotonic regulator in a concentration range from 0.5% to 10% (m/v), more preferably 1% to 5% (m/v). According to Chinese patent application CN200480034152.8 filed by Novo Nordisk, commonly used isotonic regulator such as glucose, mannitol, xylitol, fructose, lactose, maltose, sucrose, trehalose, glycerol, glycine, histidine or arginine and so on can not be used with liraglutide, which are easy to make liraglutide crystallization from the solution. After a lot of exploratory experiments, the inventors accidentally found that if the use of xylitol instead of propylene glycol and with surfactants in combination can play the both role of isotonic regulator and stabilizer to promote liraglutide solution stable, not easy to crystallize. In addition, the inventors have surprisingly found that the addition of xylitol can reduce the formation of high molecular impurities, such as dimers, in the liraglutide formulation, which is more stable than Victoza prescription. This unexpected technical effect is never reported in the existing public literature.

In the pharmaceutical compositions disclosed in the present invention, the stabilizer for improving the stability of liraglutide is preferably selected from the group consisting of polysorbate 20, polysorbate 80, poloxamer 124, poloxamer 181, poloxamer 182, poloxamer 188, poloxamer 237, poloxamer 331, poloxamer 338, poloxamer 407 or a mixture thereof, more preferably selected from polysorbate 20, polysorbate 80, poloxamer 188 or a mixture thereof.

The pharmaceutical compositions of the present invention comprises a pharmaceutically acceptable preservative. Suitable pharmaceutically acceptable preservatives may be selected from the group consisting of phenol, o-cresol, m-cresol, p-cresol, methyl p-hydroxybenzoate, propyl p-hydroxybenzoate, 2-phenoxyethanol, p-hydroxybenzene butyl formate, 2-phenylethanol, benzyl alcohol, chlorobutanol, chlorocresol, ethyl p-hydroxybenzoate or mixtures thereof in a concentration from about 0.1mg/ml to 10mg/ml, preferably in a concentration from about 1mg/ml to 8mg/ml, and most preferably in a concentration from about 2 mg/ml to 6 mg/ml.

The preferred preservative in the pharmaceutical composition of the present invention is phenol or m-cresol, which may be used singly or in combination. A more preferred preservative is phenol at a concentration in the range of about 0.1 mg/ml to about 10mg/ml, preferably at a concentration in the range from about 1mg/ml to 8mg/ml, and most preferably at a concentration in the range of about 2mg/ml to 6mg/ml.

After a lot of comparative explorations, the inventors found that polysorbate 20, polysorbate 80, poloxamer 188 or a mixture thereof at a concentration in the range of 0.001 to 0.5% (m / v) as a stabilizer combined with xylitol can effectively promote the long-term stability of liraglutide formulations and prevent liraglutide precipitating from the solution. Polysorbate and poloxamer 188 may be used alone or in combination. When used alone,the concentration of polysorbate 20, polysorbate 80 or poloxamer 188 may preferably be in the range from about 0.004% to about 0.3%(m/v), most preferably 0.02%(m/v). When used in combination, the concentration of polysorbate 80 in the range from about 0.001% to about 0.3% (m/v) and the concentration of poloxamer 188 in the range from about 0.001 % to about 0.3 % (m/v),more preferably the concentration of polysorbate 80 in the range from about 0.004% to about 0.2% (m/v) and the concentration of poloxamer 188 in the range from about 0.004% to about 0.2% (m/v),more preferably the concentration of polysorbate 80 is 0.01% (m/v) and the concentration of poloxamer 188 is 0.01%(m/v). The combination of the stabilizer and xylitol is particularly useful for preventing liraglutide precipitation from the solution and for inhibiting the formation of high molecular impurities such as dimers.

Preferably, the above pharmaceutical composition comprises liraglutide at a concentration from 0.1mg/ml to 25mg/ml , pH from 7.5 to 9.0, disodium hydrogen phosphate buffer at a concentration from 5 mmol/L to 100 mmol/L, polysorbate 80 or poloxamer 188 at a concentration from 0.001% to 0.5% (m/v), xylitol at a concentration from 0.5% to 10% (m/v), phenol or m-cresol at a concentration from 0.1 mg/ml to 10 mg/ml.

Preferably, the above pharmaceutical composition comprises liraglutide at a concentration from 0.1mg/ml to 25mg/ml , pH from 7.5 to 9.0, disodium hydrogen phosphate buffer at a concentration from 5 mmol/L to 100 mmol/L, polysorbate 80 at a concentration from 0.001% to 0.5% (m/v), poloxamer 188 at a concentration from 0.001% to 0.5%(m/v), xylitol at a concentration from 0.5% to 10% (m/v), phenol or m-cresol at a concentration from 0.1 mg/ml to 10 mg/ml.

Preferably, the above pharmaceutical composition comprises liraglutide at a concentration from 1mg/ml to 15mg/ml , pH from 7.5 to 8.5, disodium hydrogen phosphate buffer at a concentration from 10 mmol/L to 30mmol/L, polysorbate 80 or poloxamer 188 at a concentration from 0.004% to 0.3% (m/v), xylitol at a concentration from 1% to 5% (m/v),phenol or m-cresol at a concentration from 1 mg/ml to 8mg/ml.

Preferably, the above pharmaceutical composition comprises liraglutide at a concentration from 1mg/ml to 15mg/ml, pH from 7.5 to 8.5, disodium hydrogen phosphate buffer at a concentration from 10 mmol/L to 30mmol/L, polysorbate 80 at a concentration from 0.004% to 0.3% (m/v), poloxamer 188 at a concentration from 0.004% to 0.3% (m/v), xylitol at a concentration from 1% to 5% (m/v), phenol or m-cresol at a concentration from 1 mg/ml to 8mg/ml.

Preferably, the above pharmaceutical composition comprises liraglutide at a concentration from 3mg/ml to 10mg/ml, pH from 8.0 to 8.5, disodium hydrogen phosphate buffer at a concentration from 10mmol/L to 30mmol/L, polysorbate 80 or poloxamer 188 at a concentration 0.02% (m/v), xylitol at a concentration from 1% to 5% (m/v),phenol or m-cresol at a concentration from 2 mg/ml to 6 mg/ml.

Preferably, the above pharmaceutical composition comprises liraglutide at a concentration from 3mg/ml to 10mg/ml, pH from 8.0 to 8.5, disodium hydrogen phosphate buffer at a concentration from 10 mmol/L to 30mmol/L, polysorbate 80 at a concentration 0.01%(m/v), poloxamer 188 at a concentration 0.01% (m/v), xylitol at a concentration from 1% to 5% (m/v), phenol or m-cresol at a concentration from 2 mg/ml to 6mg/ml.

If necessary, the above pharmaceutical composition can be prepared as a lyophilized powder, and the lyophilized powder can be reconstituted to the state of the solution before lyophilization by adding pharmaceutically acceptable diluent, for example, by adding water for injection to dissolve. Lyophilization can be carried out using commonly techniques in the art, for example, freeze-drying cycles include freezing, primary drying and secondary drying. Since the liquid preparations before lyophilization are substantially isotonic and/or iso-osmolar, so the lyophilized powder can be reconstituted to be the isotonic or iso-osmolar solutions by adding an appropriate amount of water for injection.

The above pharmaceutical compositions are useful for the treatment of patients with type 2 diabetes mellitus for improving blood glucose control, either alone or in combination with metformin, sulfonylureas and so on. The pharmaceutical composition is administrated for subcutaneous injection,one of the preferred dosing regimens is once daily, 0.6 mg/day for the first week and 1.2 mg/day thereafter; if the 1.2 mg dose does not significantly control blood glucose, the dose is increased to 1.8 mg/day.

The said method for preparing a liraglutide pharmaceutical composition, wherein the preservative, the buffer and the stabilizer are defined as described above, and the preferred liraglutide pharmaceutical composition is also as described above.

The solution obtained in step (3) can be filtered with a 0.22 µm filter ,and then can be used further for preparing formulation.

The following examples are provided for clarity of the present invention, and are merely illustrative of the present invention and are not intended to be limiting.

### EXAMPLES

### Example 1

### Investigating the dissolution of liraglutide powder at different pH

The appropriate amount of liraglutide powder was dissolved in the water for injection and the disodium hydrogen phosphate buffer at different pH, the dissolution shown in Table 1.

**Table 1 The dissolution of the liraglutide powder in the solution at different pH**

| | The concentration of liraglutide | Dissolution |
|---|---|---|
| water for injection | 6mg/ml | insoluble |
| 10mM disodium hydrogen phosphate buffer (pH7.00) | 6mg/ml | insoluble |
| 10mM disodium hydrogen phosphate buffer (pH7.50) | 6mg/ml | colorless and clear |
| 10mM disodium hydrogen phosphate buffer (pH8.00) | 6mg/ml | colorless and clear |
| 10mM disodium hydrogen phosphate buffer (pH8.15) | 6mg/ml | colorless and clear |
| 10mM disodium hydrogen phosphate buffer (pH8.50) | 6mg/ml | colorless and clear |
| 10mM disodium hydrogen phosphate buffer (pH9.00) | 6mg/ml | colorless and clear |

From the above test we could know: liraglutide powder didn't dissolve in acidic and neutral conditions,and were dissolved in the alkaline conditions.

### Example 2

### Investigating the osmotic pressure of the solution containing different isotonic agent

The isotonic agent was dissolved in 10 mM disodium phosphate buffer and liraglutide was added to 6 mg/ml with stirring and the pH was adjusted to pH 8.15 with sodium hydroxide. Finally, the solution was filtered through a 0.22 µm filter, respectively. The concentration of each solution isotonic agent and osmotic pressure test results shown in Table 2:

**Table 2 Concentration of isotonic agent and osmotic pressure test results**

| Isotonic agent | Osmotic pressure |
|---|---|
| Negative control (no isotonic agent) | 0.041 |
| Methionine (15 mg/ml) | 0.141 |
| Glycine (15 mg/ml) | 0.301 |
| Xylitol (28 mg/ml) | 0.284 |
| PEG400 (61 mg/ml) | 0.291 |
| L-arginine (25 mg/ml) | 0.322 |
| Sorbitol (32 mg/ml) | 0.277 |
| Glycerol (16.8 mg/ml) | 0.289 |
| Sodium chloride (8.6 mg/ml) | 0.307 |
| Victoza | 0.281 |

The isotonic solution had a osmolality of about 0.285 to 0.310 osmol / L.

### Example 3

### Examining the stability of the formulation solution containing different stabilizers

The preservatives, isotonic agents and buffers were dissolved in water for injection and the liraglutide powder was dissolved in the solution with slow stirring and then the pH is adjusted to the desired pH with sodium hydroxide and / or hydrochloric acid, then certain amount of stabilizer was added. Finally, the above formulation solution was filtered through a 0.22 µm filter. The type and amount of stabilizer added were shown in Table 3.

The composition of the formulation was as follows:
Liraglutide: 6mg / ml
Disodium hydrogen phosphate: 1.42mg / ml
Phenol: 5.5mg / ml
Isotonic agent: appropriate amount
Stabilizer: appropriate amount
Water for injection: to 1 ml
PH: 8.15

**Table 3, the type,amount of stabilizer and isotonic agents**

| No. | Isotonic agents | Concentration | Stabilizer | Concentration |
|---|---|---|---|---|
| 1 | Xylitol | 28mg/ml | Polysorbate 80 | 0.02% |
| 2 | Xylitol | 28mg/ml | Poloxamer 188 | 0.02% |
| 3 | Xylitol | 28mg/ml | Polysorbate 80 + Poloxamer 188 | 0.01% +0.01 % |
| 4 | Sodium chloride | 8mg/ml | Polysorbate 80 + Poloxamer 188 | 0.01% +0.01 % |
| 5 | Xylitol | 28mg/ml | Hydroxypropyl-β-cyclodextrin | 2% |
| 6 | Xylitol | 28mg/ml | PovidoneK 30 | 3% |
| 7 | Xylitol | 28mg/ml | PEG300 | 3% |
| 8 | - | - | Victoza prescription (including propylene glycol) | 14mg/ml |

The above preparations were incubated at 37°C, 25°C and 4°C for stability investigation, and the related substances (area normalization) of the samples were detected by HPLC. The results were shown as follows:

**Table 4 Stability test at 37°C**

| | Formulation No. | | | | | | | |
|---|---|---|---|---|---|---|---|---|
| Time | 1 | 2 | 3 | 4 | 5 | 6 | 7 | 8 |
| Start time | 0.17% | 0.18% | 0.20% | 0.18% | 2.48% | 0.14% | 0.18% | 0.50% |
| 1 week | 1.38% | 0.70% | 1.66% | 1.63% | 2.59% | 27.6% | 4.24% | 1.58% |
| 2 week | 1.52% | 1.47% | 2.03% | 2.13% | 6.00% | 69.31 % | 5.15% | 1.72% |
| 3 week | 3.77% | 3.85% | 4.56% | 4.46% | 7.41% | ----- | 6.73% | 4.73% |
| 4 week | 3.51% | 3.33% | 4.18% | 4.63% | 9.23% | ----- | 6.34% | 4.29% |

**Table 5 Stability test at 25 °C**

| | Formulation No. | | | | | | | |
|---|---|---|---|---|---|---|---|---|
| Time | 1 | 2 | 3 | 4 | 5 | 6 | 7 | 8 |
| Start time | 0.17% | 0.18% | 0.20% | 0.18% | 2.48% | 0.14% | 0.18% | 0.50% |
| 1 week | 0.84% | 0.80% | 0.85 % | 0.91% | 3.19% | 21.16 % | 2.41 % | 2.63% |
| 2 week | 1.33 % | 1.20% | 1.99% | 1.99% | 3.82% | 64.26 % | 4.29% | 1.99 % |
| 3 week | 1.92% | 2.07% | 1.94% | 2.12% | 5.04% | ----- | 4.97% | 2.22 % |
| 4 week | 2.22% | 2.19% | 2.32 % | 2.57% | 6.77% | ----- | 5.50% | 2.76% |

**Table 6 Stability test at 4°C**

| Time | Formulation No. | | | | | | | |
|---|---|---|---|---|---|---|---|---|
| | 1 | 2 | 3 | 4 | 5 | 6 | 7 | 8 |
| Start time | 0.17% | 0.18% | 0.20% | 0.18% | 2.48% | 0.14% | 0.18% | 0.50% |
| 2 week | 0.31% | 0.48% | 0.68% | 0.59% | 3.05% | 16.84 % | 1.53% | 1.02 % |
| 4 week | 0.49% | 0.80% | 1.18% | 1.11% | 3.55% | ----- | 2.15% | 1.69% |

From the above results we could see that low concentration of polysorbate 80 or poloxamer 188 can effectively increase the stability of liraglutide preparation, even in the accelerated tests can maintain the purity of the preparation more than 95%, its ability of increasing the prepararion stability was better than hydroxypropyl-β-cyclodextrin, povidoneK30 or PEG300 et al., even better than the stability of the imported Victoza prescription. During the stability study period No.1-4 preparation solution was clear, no visible foreign matter nor crystallization phenomenon was observed.

### Example 4

### Investigating formulation stability with different concentrations of poloxamer 188 as stabilizer

The preservatives, isotonic agents and buffers are dissolved in water for injection and the liraglutide powder was dissolved in the solution with slow stirring and then the pH is adjusted to the desired pH with sodium hydroxide and / or hydrochloric acid, then certain amount of poloxamer 188 was added. Finally, the above formulation solution was filtered through a 0.22 µm filter. The amount of poloxamer 188 added were shown in Table 7.

The composition of the preparation was as follows:
Liraglutide: 6mg / ml
Disodium hydrogen phosphate: 1.42mg / ml
Phenol: 5.5mg / ml
Xylitol :28.0mg / ml
Poloxamer 188: prescription dosage
Water for injection: to 1 ml
PH: 8.15

**Table 7 poloxamer 188 concentration of different prescriptions**

| Formulation No. | Poloxamer 188 dosage |
|---|---|
| 1 | Victoza prescription |
| 2 | 0 |
| 3 | 0.004% |
| 4 | 0.01% |
| 5 | 0.02 % |
| 6 | 0.03% |
| 7 | 0.04% |
| 8 | 0.05% |
| 9 | 0.1 % |
| 10 | 0.2% |

The above preparations were incubated at 37°C, 25°C and 4°C for stability study.The related substances of the samples such as degradation products were detected by RP-HPLC (area normalization), and high molecular impurities such as polymers or / and dimers were detected by SEC-HPLC (area normalization). During the stability study, the solution of each numbered group was clear, and no visible foreign matter nor crystallization phenomenon was observed.The results were shown as follows:

**Table 8 The related substances results of stability test at 37°C**

| No. | Time | | | | |
|---|---|---|---|---|---|
| | Start time | 1 month | 2 month | 3 month | 6 month |
| 1 | 0.88% | 4.06% | 15.78% | 22.38% | 31.56% |
| 2 | 0.92% | 5.71% | 19.74% | 19.39% | 31.67% |
| 3 | 0.92% | 3.32% | 11.05% | 20.27% | 33.88% |
| 4 | 0.92% | 3.28% | 10.96% | 19.37% | 32.06% |
| 5 | 0.90% | 3.46% | 11.09% | 18.96% | 32.67% |
| 6 | 0.89% | 3.56% | 11.62% | 19.67% | 34.22% |
| 7 | 0.88% | 3.49% | 11.53% | 20.32% | 34.55% |
| 8 | 0.91% | 3.58% | 12.79% | 20.52% | 35.17% |
| 9 | 0.88% | 3.75% | 12.15% | 2090% | 33.73% |
| 10 | 0.91 % | 3.61% | 11.70% | 20.97% | 33.69% |

**Table 9 The related substances results of stability test at 25 °C**

| No. | Time | | | | |
|---|---|---|---|---|---|
| | Start time | 1 month | 2 month | 3 month | 6 month |
| 1 | 0.88% | 1.52% | 3.24% | 4.63% | 6.39% |
| 2 | 0.92% | 3.42% | 5.82% | 3.53% | 6.39% |
| 3 | 0.92% | 1.43% | 3.01% | 3.83% | 6.66% |
| 4 | 0.92% | 1.45% | 2.93% | 3.83% | 6.73% |
| 5 | 0.90% | 1.46% | 2.56% | 3.87% | 6.58% |
| 6 | 0.89% | 1.51% | 2.60% | 3.79% | 6.47% |
| 7 | 0.88% | 1.50% | 2.95% | 3.95% | 6.60% |
| 8 | 0.91% | 1.55% | 3.06% | 4.08% | 6.69% |
| 9 | 0.88% | 2.43% | 3.03% | 4.14% | 6.78% |
| 10 | 0.91% | 2.50% | 3.11% | 4.13% | 7.04% |

**Table 10 The related substances results of stability test at 4°C**

| No. | Time | | | | |
|---|---|---|---|---|---|
| | Start time | 3 month | 6 month | 9 month | 12 month |
| 1 | 0.88% | 1.33% | 2.11% | 1.28% | 3.17% |
| 2 | 0.92% | 1.63% | 1.10% | 1.20% | 2.96% |
| 3 | 0.92% | 1.09% | 1.11% | 1.02% | 2.81% |
| 4 | 0.92% | 1.09% | 1.15% | 0.99% | 2.74% |
| 5 | 0.90% | 1.03% | 1.07% | 0.97% | 3.07% |
| 6 | 0.89% | 1.04% | 1.05% | 0.95% | 2.97% |
| 7 | 0.88% | 1.04% | 1.06% | 0.96% | 3.21% |
| 8 | 0.91% | 1.05% | 1.08% | 0.96% | 2.77% |
| 9 | 0.88% | 0.98% | 1.08% | 0.95% | 2.86% |
| 10 | 0.91% | 1.14% | 1.13% | 1.00% | 3.35% |

**Table 11 High molecular impurities results of stability test at 37°C**

| No. | Time | | | | |
|---|---|---|---|---|---|
| | Start time | 1 month | 2 month | 3 month | 6 month |
| 1 | 0.45% | 1.01% | 3.70% | 4.93% | 8.25% |
| 2 | 0.09% | 1.84% | 4.50% | 4.81% | 8.69% |
| 3 | 0.11% | 0.89% | 2.60% | 4.88% | 8.72% |
| 4 | 0.09% | 0.92% | 3.05% | 4.87% | 8.57% |
| 5 | 0.10% | 0.95% | 2.68% | 4.65% | 9.66% |
| 6 | 0.13% | 0.97% | 2.90% | 4.67% | 9.53% |
| 7 | 0.11% | 0.84% | 2.63% | 4.80% | 10.53% |
| 8 | 0.12% | 0.89% | 2.70% | 4.83% | 9.04% |
| 9 | 0.12% | 1.04% | 2.86% | 4.31% | 9.60% |
| 10 | 0.13% | 1.01% | 2.54% | 4.63% | 9.57% |

**Table 12 High molecular impurities results of stability test at 25 °C**

| No. | Time | | | | |
|---|---|---|---|---|---|
| | Start time | 1 month | 2 month | 3 month | 6 month |
| 1 | 0.45% | 0.61% | 1.04% | 1.34% | 2.10% |
| 2 | 0.09% | 0.90% | 1.51% | 1.08% | 2.22% |
| 3 | 0.11% | 0.49% | 0.83% | 1.15% | 1.95% |
| 4 | 0.09% | 0.42% | 0.88% | 1.09% | 2.06% |
| 5 | 0.10% | 0.57% | 0.81% | 1.08% | 1.90% |
| 6 | 0.13% | 0.57% | 0.87% | 1.11% | 1.95% |
| 7 | 0.11% | 0.50% | 0.86% | 1.12% | 2.07% |
| 8 | 0.12% | 0.62% | 0.90% | 1.23% | 2.06% |
| 9 | 0.12% | 0.54% | 0.80% | 1.18% | 2.08% |
| 10 | 0.13% | 0.61% | 0.88% | 1.23% | 2.05% |

**Table 13 High molecular impurities results of stability test at 4°C**

| No. | Time | | | | |
|---|---|---|---|---|---|
| | Start time | 3 month | 6 month | 9 month | 12 month |
| 1 | 0.45% | 0.32% | 0.37% | 0.56% | 1.30% |
| 2 | 0.09% | 0.24% | 0.35% | 0.50% | 1.40% |
| 3 | 0.11% | 0.30% | 0.26% | 0.55% | 0.87% |
| 4 | 0.09% | 0.30% | 0.38% | 0.50% | 1.08% |
| 5 | 0.10% | 0.30% | 0.38% | 0.54% | 1.10% |
| 6 | 0.13% | 0.35% | 0.35% | 0.56% | 1.14% |
| 7 | 0.11% | 0.31% | 0.41% | 0.53% | 1.35% |
| 8 | 0.12% | 0.32% | 0.33% | 0.56% | 1.13% |
| 9 | 0.12% | 0.28% | 0.33% | 0.49% | 1.23% |
| 10 | 0.13% | 0.39% | 0.32% | 0.60% | 1.30% |

From the above results we could see that the physical stability and chemical stability of the liraglutide preparation containing 0.004% -0.03% poloxamer 188 and isotonic agent xylitol were significantly increased. Stored at 4°C for 12 months, the purity of liraglutide was still remained more than 97%, and the preparation solution remained clear, no visible foreign matter nor crystallization lead to syringe needle clogging phenomenon were observed. Especially for stability test at 4°C, 25°C and 37°C,the results of high molecular impurities were better than the imported Victoza prescription.

### Embodiments:

1. A pharmaceutical formulation comprising liraglutide in a concentration from 0.1mg/ml to 25mg/ml, a buffer with pH from 7.5 to 9.0, a stabilizer in a concentration from 0.001% to 0.5% (m/v), xylitol in a concentration from 0.5% to 10%(m/v), and preservative in a concentration from 0. 1mg/ml to 10 mg/ml.
2. The formulation according embodiment 1, wherein said liraglutide is present in a concentration from 1mg/ml to 15mg/ml.
3. The formulation according embodiment 2, wherein said liraglutide is present in a concentration from 3mg/ml to 10mg/ml.
4. The formulation according embodiment 3, wherein said liraglutide is present in a concentration 6mg/ml.
5. The formulation according embodiment 1, wherein said buffer is any buffer capable of maintaining the pH of the formulation in the aqueous solution at a pH from 7.5 to 9.0, which can be selected from phosphate buffer, a disodium hydrogen phosphate-Citrate buffer,TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof.
6. The formulation according embodiment 5, wherein said buffer is at a pH from 7.5 to 8.5.
7. The formulation according embodiment 6, wherein said buffer is at a pH from 8.0 to 8.5.
8. The formulation according embodiment 5, wherein said buffer is in a concentration from 5mmol/L to 100mmol/L.
9. The formulation according embodiment 8, wherein said buffer is in a concentration from 10mmol/L to 30mmol/L.
10. The formulation according embodiment 5, wherein said buffer is sodium dihydrogen phosphate buffer in a concentration from 5mmol/L to 100mmol/L, and at a pH from 7.5 to 8.5.
11. The formulation according embodiment 10, wherein said buffer is sodium dihydrogen phosphate buffer in a concentration from 10mmol/L to 30mmol/L, and at a pH from 8.0 to 8.5.
12. The formulation according embodiment 1, wherein said xylitol is present in a concentration from 1% to 5%(m/v).
13. The formulation according embodiment 1, wherein said stabilizer is selected from polysorbate 20, polysorbate 80, poloxamer 124, poloxamer 181, poloxamer 182, poloxamer 188, poloxamer 237, poloxamer 331, poloxamer 338, poloxamer 407 or a mixture thereof.
14. The formulation according embodiment 13, wherein said stabilizer is selected from polysorbate 20, polysorbate 80 or poloxamer 188 in a concentration from 0.004% to 0.3 % (m/v).
15. The formulation according embodiment 14, wherein said stabilizer is selected from polysorbate 20, polysorbate 80 or poloxamer 188 in a concentration 0.02% (m/v).
16. The formulation according embodiment 1, wherein said stabilizer is a mixture of polysorbate 80 and poloxamer 188 ,comprising polysorbate 80 in a concentration from 0.001 % to 0.3 %(m/v) and poloxamer 188 in a concentration from 0.001 % to 0.3 %(m/v).
17. The formulation according embodiment 16, wherein said stabilizer is a mixture of polysorbate 80 and poloxamer 188 ,comprising polysorbate 80 in a concentration from 0.004% to 0.2 % (m/v) and poloxamer 188 in a concentration from 0.004% to 0.2 % (m/v).
18. The formulation according embodiment 16, wherein said stabilizer is a mixture of polysorbate 80 and poloxamer 188 ,comprising polysorbate 80 in a concentration 0.01% (m/v) and poloxamer 188 in a concentration 0.01 % (m/v).
19. The formulation according embodiment 1, wherein said preservative is selected from phenol, o-cresol, m-cresol, p-cresol, methyl p-hydroxybenzoate, propyl p-hydroxybenzoate, 2-phenoxyethanol, p-hydroxybenzene butyl formate, 2-phenylethanol, benzyl alcohol, chlorobutanol, chlorocresol, ethyl p-hydroxybenzoate or mixtures thereof , in a concentration from about 0.1 mg/ml to 10mg/ml.
20. The formulation according embodiment 19, wherein said preservative is present in a concentration from about 1mg/ml to 8mg/ml.
21.The formulation according embodiment 20, wherein said preservative is present in a concentration from about 2mg/ml to 6mg/ml.
22. The formulation according any one of embodiments 19-21, wherein said preservative is phenol or m-cresol, which may be used singly or in combination.
23. The formulation according embodiment 19, wherein said preservative is phenol in a concentration from about 0.1mg/ml to 10mg/ml.
24. The formulation according embodiment 23, wherein said preservative is phenol in a concentration from about 1mg/ml to 8mg/ml.
25. The formulation according embodiment 24, wherein said preservative is phenol in a concentration from about 2mg/ml to 6mg/ml.
26. A pharmaceutical formulation comprising liraglutide at a concentration from 0. 1mg/ml to 25mg/ml , pH from 7.5 to 9.0, disodium hydrogen phosphate buffer at a concentration from 5 mmol/L to 100 mmol/L, polysorbate 80 or poloxamer 188 at a concentration from 0.001% to 0.5% (m/v), xylitol at a concentration from 0.5% to 10% (m/v),phenol or m-cresol at a concentration from 0.1 mg/ml to 10 mg/ml.
27. A pharmaceutical formulation comprising liraglutide at a concentration from 0. 1mg/ml to 25mg/ml , pH from 7.5 to 9.0, disodium hydrogen phosphate buffer at a concentration from 5 mmol/L to 100 mmol/L, polysorbate 80 at a concentration from 0.001% to 0.5% (m/v), poloxamer 188 at a concentration from 0.001% to 0.5%(m/v), xylitol at a concentration from 0.5% to 10% (m/v),phenol or m-cresol at a concentration from 0.1 mg/ml to 10 mg/ml.
28. A pharmaceutical formulation comprising liraglutide at a concentration from 1mg/ml to 15mg/ml, pH from 7.5 to 8.5, disodium hydrogen phosphate buffer at a concentration from 10mmol/L to 30 mmol/L, polysorbate 80 or poloxamer 188 at a concentration from 0.004% to 0.3% (m/v), xylitol at a concentration from 1% to 5% (m/v),phenol or m-cresol at a concentration from 1mg/ml to 8mg/ml.
29. A pharmaceutical formulation comprising liraglutide at a concentration from 1mg/ml to 15mg/ml , pH from 7.5 to 8.5, disodium hydrogen phosphate buffer at a concentration from 10mmol/L to 30mmol/L, polysorbate 80 at a concentration from 0.004% to 0.3% (m/v), poloxamer 188 at a concentration from 0.004% to 0.3% (m/v), xylitol at a concentration from 1% to 5% (m/v),phenol or m-cresol at a concentration from 1 mg/ml to 8mg/ml.
30. A pharmaceutical formulation comprising liraglutide at a concentration from 3mg/ml to 10mg/ml, pH from 8.0 to 8.5, disodium hydrogen phosphate buffer at a concentration from 10mmol/L to 30mmol/L, polysorbate 80 or poloxamer 188 at a concentration 0.02% (m/v), xylitol at a concentration from 1% to 5% (m/v),phenol or m-cresol at a concentration from 2 mg/ml to 6 mg/ml.
31. A pharmaceutical formulation comprising liraglutide at a concentration from 3mg/ml to 10mg/ml , pH from 8.0 to 8.5, disodium hydrogen phosphate buffer at a concentration from 10 mmol/L to 30mmol/L, polysorbate 80 at a concentration 0.01%(m/v), poloxamer 188 at a concentration 0.01% (m/v), xylitol at a concentration from 1% to 5% (m/v), phenol or m-cresol at a concentration from 2mg/ml to 6mg/ml.
32. The formulation according any one of embodiments 1-31, wherein said formulation can be prepared as a lyophilized powder.
33. Use of the formulation according any one of embodiments 1-31 for the treatment of patients with type 2 diabetes mellitus.
34. The use according embodiment 33, wherein said formulation is administrated either alone or in combination with metformin, sulfonylureas et al.
35. The use according embodiment 34, wherein said formulation is administrated for subcutaneous injection
36. The use according embodiment 35, wherein the dosing regimen of said formulation is once daily, 0.6 mg/day for the first week and 1.2 mg/day thereafter; if the 1.2 mg dose does not significantly control blood glucose, the dose is increased to 1.8 mg/day.
37.A method for preparing the liraglutide formulation comprising the following steps:
   (1) dissolving a preservative, xylitol and a buffering agent in water for injection to prepare a solution;
   (2) dissolving the liraglutide in the above solution, adjusting the pH to the desired pH;
   (3) adding a stabilizer to the above solution to obtain the pharmaceutical formulation according embodiment 1.
38.The method according embodiment 37,wherein the liraglutide in the formulation is present in a concentration from 1mg/ml to 15mg/ml.
39.The method according embodiment 38, wherein the liraglutide in the formulation is present in a concentration from 3mg/ml to 10mg/ml.
40. The method according embodiment 39, wherein the liraglutide in the formulation is present in a concentration 6mg/ml.
41. The method according embodiment 37, wherein the buffer in process step(1) is any buffer capable of maintaining the pH of the formulation in the aqueous solution at a pH from 7.5 to 9.0, which can be selected from phosphate buffer, a disodium hydrogen phosphate-Citrate buffer,TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof.
42. The method according embodiment 41, wherein the buffer in process step(1) is at a pH from 7.5 to 8.5.
43. The method according embodiment 42, wherein the buffer in process step(1) is at a pH from 8.0 to 8.5.
44. The method according embodiment 41, wherein the buffer in process step(1) is in a concentration from 5mmol/L to 100mmol/L.
45. The method according embodiment 37, wherein the buffer in process step(1) is in a concentration from 10mmol/L to 30mmol/L.
46. The method according embodiment 41, wherein the buffer in process step(1) is sodium dihydrogen phosphate buffer in a concentration from 5mmol/L to 100mmol/L, and at a pH from 7.5 to 8.5.
47. The method according embodiment 46, wherein the buffer in process step(1) is sodium dihydrogen phosphate buffer in a concentration from 10mmol/L to 30mmol/L, and at a pH from 8.0 to 8.5.
48. The method according embodiment 37, wherein the xylitol in the formulation is present in a concentration from 1% to 5%(m/v).
49. The method according embodiment 37, wherein the preservative in process step(1) is selected from phenol, o-cresol, m-cresol, p-cresol, methyl p-hydroxybenzoate, propyl p-hydroxybenzoate, 2-phenoxyethanol, p-hydroxybenzene butyl formate, 2-phenylethanol, benzyl alcohol, chlorobutanol, chlorocresol, ethyl p-hydroxybenzoate or mixtures thereof, the concentration of the preservative in the formulation is from about 0.1mg/ml to 10mg/ml.
50. The method according embodiment 49, wherein the concentration of the preservative in the formulation is from about 1mg/ml to 8mg/ml.
51. The method according embodiment 50, wherein the concentration of the preservative in the formulation is from about 2mg/ml to 6mg/ml.
52. The method according any one of embodiments 49-51, wherein said preservative is phenol or m-cresol.
53. The method according embodiment 52, wherein the preservative in the formulation is phenol, the concentration is from about 0.1mg/ml to 10mg/ml.
54. The method according embodiment 53, wherein the preservative in the formulation is phenol, the concentration is from about 1mg/ml to 8mg/ml.
55. The method according embodiment 54, wherein the preservative in the formulation is phenol, the concentration is from about 2mg/ml to 6mg/ml.
56. The method according embodiment 37, wherein the stabilizer in process step(3) is selected from polysorbate 20, polysorbate 80, poloxamer 124, poloxamer 181, poloxamer 182, poloxamer 188, poloxamer 237, poloxamer 331, poloxamer 338, poloxamer 407 or a mixture thereof.
57. The method according embodiment 56, wherein the stabilizer in process step(3) is selected from polysorbate 20, polysorbate 80 or poloxamer 188 in a concentration from 0.004% to 0.3% (m/v).
58. The method according embodiment 57, wherein the stabilizer in process step(3) is selected from polysorbate 20, polysorbate 80 or poloxamer 188 in a concentration 0.02% (m/v).
59. The method according embodiment 58, wherein the stabilizer in process step(3) is a mixture of polysorbate 80 and poloxamer 188 ,comprising polysorbate 80 in a concentration from 0.001 % to 0.5 %(m/v) and poloxamer 188 in a concentration from 0.001% to 0.5% (m/v).
60. The method according embodiment 59, wherein the stabilizer in process step(3) is a mixture of polysorbate 80 and poloxamer 188 ,comprising polysorbate 80 in a concentration from 0.004% to 0.3%(m/v) and poloxamer 188 in a concentration from 0.004% to 0.3% (m/v).
61. The method according embodiment 60, wherein the stabilizer in process step(3) is a mixture of polysorbate 80 and poloxamer 188 ,comprising polysorbate 80 in a concentration 0.01 %(m/v) and poloxamer 188 in a concentration 0.01 %(m/v).
62. The method according embodiment 37, wherein the prepared formulation comprising liraglutide at a concentration from 1mg/ml to 15mg/ml , pH from 7.5 to 8.5, disodium hydrogen phosphate buffer at a concentration from 10mmol/L to 30 mmol/L, polysorbate 80 or poloxamer 188 at a concentration from 0.004% to 0.3% (m/v), xylitol at a concentration from 1% to 5% (m/v), phenol or m-cresol at a concentration from 1mg/ml to 8mg/ml.
63. The method according embodiment 37, wherein the prepared formulation comprising liraglutide at a concentration from 1mg/ml to 15mg/ml , pH from 7.5 to 8.5, disodium hydrogen phosphate buffer at a concentration from 10mmol/L to 30mmol/L, polysorbate 80 at a concentration from 0.004% to 0.3% (m/v), poloxamer 188 at a concentration from 0.004% to 0.3% (m/v), xylitol at a concentration from 1% to 5% (m/v), phenol or m-cresol at a concentration from 1 mg/ml to 8mg/ml.
64. The method according embodiment 37, wherein the prepared formulation comprising liraglutide at a concentration from 3mg/ml to 10mg/ml , pH from 8.0 to 8.5, disodium hydrogen phosphate buffer at a concentration from 10mmol/L to 30mmol/L, polysorbate 80 or poloxamer 188 at a concentration 0.02% (m/v), xylitol at a concentration from 1% to 5% (m/v), phenol or m-cresol at a concentration from 2 mg/ml to 6 mg/ml.
65. The method according embodiment 37, wherein the prepared formulation comprising liraglutide at a concentration from 3mg/ml to 10mg/ml , pH from 8.0 to 8.5, disodium hydrogen phosphate buffer at a concentration from 10 mmol/L to 30mmol/L, polysorbate 80 at a concentration 0.01%(m/v), poloxamer 188 at a concentration 0.01% (m/v), xylitol at a concentration from 1% to 5% (m/v), phenol or m-cresol at a concentration from 2mg/ml to 6mg/ml.
66.A pharmaceutical formulation comprising a GLP-1 analog in a concentration from 0. 1mg/ml to 25mg/ml, a buffer with pH from 7.5 to 9.0, a stabilizer in a concentration from 0.001% to 0.5% (m/v), xylitol in a concentration from 0.5% to 10%(m/v), and a preservative in a concentration from 0.1 mg/ml to 10 mg/ml;
   Wherein the GLP-1 analog is GLP-1 or its mutant, GLP-1 (7-3 6)-amide or its mutant, GLP-1 (7-37) or its mutant, GLP-1 derivatives which is chemically modified by introducing an organic substituent e.g. ester, alkyl or lipophilic group on one or more amino acid residues of the above peptide or its mutant thereof.
   Wherein the mutant means the parent peptide GLP-1, GLP-1 (7-36) -amide or GLP-1 (7-37) wherein one or more amino acid residues of the parent peptide have been substituted by another amino acid residue and/or wherein one or more amino acid residues of the parent peptide have been deleted and/or wherein one or more amino acid residues have been added to the parent peptide.
67.The formulation according embodiment 66,wherein the lipophilic substituent attached to the GLP-1 derivative has 4-40 carbon atoms, more preferably 8-30 carbon atoms, even more preferably 8-25 carbon atoms, even more preferably 12-25 carbon atoms, and most preferably 14-18 carbon atoms.
68. The formulation according embodiment 66, wherein the GLP-1 analog is Arg³⁴,Lys²⁶(N^{ε}-(γ-Glu(N^{α}-hexadecanoyl)))-GLP-1(7-37).
69. The formulation according embodiment 66, wherein the GLP-1 analog is selected from Gly⁸-GLP-1(7-36)-amide, Gly⁸-GLP-1(7-37), Val⁸-GLP-1(7-36)-amide, Val⁸-GLP-1(7-37), Val⁸Asp²²-GLP-1(7-37), Val⁸Asp²²-GLP-1(7-36)-amide, Val⁸Glu²²-GLP-1 (7-36)-amide, Val⁸Glu²²-GLP-1(7-37), Val⁸Lys²²-GLP-1(7-36)-amide, Val⁸Lys²²-GLP-1(7-37), Val⁸Arg²²-GLP-1(7 -36)-amide, Val⁸Arg²²-GLP-1 (7-37), Val⁸His²²-GLP-1(7-36)-amide, Val⁸His²²-GLP-1(7-37); Arg²⁶-GLP-1(7-37); Arg³⁴-GLP-1(7-37); Lys³⁶-GLP-1(7-37); Arg^{26, 34}Lys³⁶-GLP-1(7-37); Arg^{26, 34}-GLP-1(7-37); Arg^{26, 34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly^{g}His²²-GLP-1(7-37); Val⁸His²²-GLP-1(7-37); Met⁸His²²-GLP-1 (7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Val⁸Asp²²-GLP-1(7-37); Met⁸Asp²²-GLP-1(7-37); GlylGlu²²-GLP-1(7-37); Val⁸Glu²²-GLP-1(7-37); Met⁸Glu²²-GLP-1(7-37); Gly^{g}Lys²²-GLP-1(7-37); Val⁸Lys²²-GLP-1(7-37); Met⁸Lys²²-GLP-1(7-37); Gly⁸Arg²²-GLP-1(7-37); Val⁸Lys²²His³⁷-GLP-1 (7-37); Gly⁸Glu²²His³⁷-GLP-1(7-37); Val⁸Glu²²His³⁷-GLP-1 (7-37); Met⁸Glu²²His³⁷-GLP-1 (7-37); Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1(7-37); Val⁸His²²His³⁷-GLP-1(7-37); Met⁸His³³His³⁷-GLP-1(7-37); Gly⁸His³⁷-GLP-1(7-37); Val⁸His³⁷-GLP-1(7-37); Met⁸His³⁷-GLP-1(7-37); Gly⁸Asp²²His³⁷-GLP-1(7-37); Val⁸Asp²²His³⁷-GLP-1(7-37); Met⁸Asp²²His³⁷-GLP-1(7-37); Arg²⁶-GLP-1(7-36)-amide; Arg³⁴-GLP-1(7-36)-amide; Lys³⁶-GLP-1(7-36)-amide; Arg^{26, 34}Lys³⁶-GLP-1(7-36)-amide; Ar^{26, 34}-GLP-1(7-36)-amide; Arg^{26, 34}Lys⁴⁰-GLP-1(7-36)-amide; Arg²⁶Lys³⁶-GLP-1(7-36)-amide; Arg³⁴Lys³⁶-GLP-1(7-36)-amide; Gly⁸-GLP-1(7-36)-amide; Val⁸-GLP-1(7-36)-amide; Met⁸-GLP-1(7-36)-amide; Gly⁸Asp²²-GLP-1(7-36)-amide; Gly⁸Glu²²His³⁷-GLP-1(7-36)-amide; Val⁸Asp²²-GLP-1(7-36)-amide; Met⁸Asp²²-GLP-1(7-36)-amide; Gly⁸Glu²²-GLP-1(7-36)-amide; Val⁸Glu²²-GLP-1(7-36)-amide; Met⁸Glu²²-GLP-1(7-36)-amide; Gly⁸Lys²²-GLP-1(7-36)-amide; Val⁸Lys²²-GLP-1(7-36)-amide; Met⁸Lys²²-GLP-1(7-36)-amide; Gly⁸His²²His³⁷-GLP-1(7-36)-amide; Gly⁸Arg²²-GLP-1(7-36)-amide; Val⁸Arg²²-GLP-1(7-36)-amide; Met⁸Arg²²-GLP-1(7-36)-amide; Gly⁸His²²-GLP-1(7-36)-amide; Val⁸His²²-GLP-1(7-36)-amide; Met⁸His²²-GLP-1(7-36)-amide; His³⁷-GLP-1(7-36)-amide; Val⁸Arg²²His³⁷-GLP-1(7-36)-amide; Met⁸Arg²²His³⁷-GLP-1(7-36)-amide; Gly⁸His³⁷-GLP-1(7-36)-amide; Val⁸His³⁷-GLP-1(7-36)-amide; Met⁸His³⁷-GLP-1(7-36)-amide; GlyAsp²²His³⁷-GLP-1(7-36)-amide; Val⁸Asp²²His³⁷-GLP-1(7-36)-amide; Met⁸Asp²²His³⁷-GLP-1(7-36)-amide; Val⁸Glu²²His³⁷-GLP-1(7-36)-amide; Met⁸Glu²²His³⁷-GLP-1(7-36)-amide; Gly⁸Lys²²His³⁷-GLP-1(7-36)-amide; Val⁸Lys²²His³⁷-GLP-1(7-36)-amide; Met⁸Lys²²His³⁷-GLP-1(7-36)-amide; Gly⁸Arg²²His³⁷-GLP-1(7-36)-amide; Val⁸His²²His³⁷-GLP-1(7-36)-amide; Met⁸His²²His³⁷-GLP-1(7-36)-amide; Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷GLP-1(7-37), Val⁸Glu²²Ile³³-GLP-1(7-37), Val⁸Trp¹⁶Glu²²Val²⁵Ile³³-GLP-1(7-37), Val⁸Trp¹⁶G1u²²Ile³³-GLP-1(7-37), Val⁸Glu²²Val²⁵Ile³³-GLP-1(7-37), Val⁸Trp¹⁶Glu²²Val²⁵-GLP-1(7-37), analogs thereof and derivatives of any of these.
70. The formulation according embodiment 66, wherein the concentration of the GLP-1 analog is from about 1mg/ml to 15mg/ml, more preferably from 3mg/ml to 10mg/ml, and most preferably 6mg/ml.
71. The formulation according embodiment 66, wherein said buffer is any buffer capable of maintaining the pH of the formulation in the aqueous solution at a pH from 7.5 to 9.0, which can be selected from phosphate buffer, a disodium hydrogen phosphate-Citrate buffer,TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof.
72. The formulation according embodiment 71, wherein the pH of said buffer is from 7.5 to 8.5, more preferably from 8.0 to 8.5.
73. The formulation according embodiment 71, wherein the concentration of said buffer is from 5mg/ml to 100mg/ml, and more preferably from 10mg/ml to 30mg/ml.
74. The formulation according embodiment 73, wherein the buffer is disodium hydrogen phosphate buffer in a concentration from 5 mmol/L to 100mmol/L ,and the pH is in the range of 7.5 to 8.5;
75. The formulation according embodiment 66, wherein said xylitol is present in a concentration from 1% to 5%(m/v).
76. The formulation according embodiment 66, wherein the stabilizer is selected from amino acids and derivatives: glycine, alanine, serine, aspartic acid, glutamic acid, threonine, tryptophan, lysine, hydroxy lysine, histidine, arginine, cystine, cysteine, methionine, phenylalanine, leucine, isoleucine Amino acids and their derivatives; nonionic surfactants: sorbitan fatty acid esters, glycerol fatty acid esters (e.g., sorbitan monoate, sorbitan monolaurate and sorbitan palm Acid monoester), polyglycerol fatty acid esters (e.g., glyceryl octanoic acid monoester, glyceryl myristate mono-tallow cream and glycerol hard fatty acid monoester), polyoxyethylene sorbitan fatty acid esters, polyoxyethylene sorbitol fatty acid esters , Polyoxyethylene glycerol fatty acid esters, polyoxyethylene glycol fatty acid esters, polyoxyethylene alkyl ethers, polyoxyethylene polyoxypropylene alkyl ethers, polyoxyethylene phenyl ethers, polyoxyethylated hard castor oil, Polyoxyethylated beeswax derivatives, polyoxyethylenated lanolin derivatives Or a polyoxyethylene fatty acid amide, the cationic surfactant is an alkyl sulfate (e.g., a C10-C18 alkyl alkyl sulfate); polyethylene glycol, polyvinyl alcohol, hydroxypropyl- Dextrins, carboxymethylcellulose, polyvinylpyrrolidone, polysorbate 20 or polysorbate 80, poloxamer series (poloxamer 124, poloxamer 181, poloxamer 182, poloxamer 188, poloxamer 237, poloxamer 331, poloxamer 338 or poloxamer 407).
77. The formulation according embodiment 76, wherein the stabilizer is selected from polysorbate 20, polysorbate 80, poloxamer 124, poloxamer 181, poloxamer 182, poloxamer 188, poloxamer 237, poloxamer 331, poloxamer 338, poloxamer 407 or a mixture thereof.
78. The formulation according embodiment 77, wherein the stabilizer is selected from polysorbate 20, polysorbate 80, poloxamer 188 or a mixture thereof.
79. The formulation according embodiment 66, wherein said preservative is selected from phenol, o-cresol, m-cresol, p-cresol, methyl p-hydroxybenzoate, propyl p-hydroxybenzoate, 2-phenoxyethanol, p-hydroxybenzene butyl formate, 2-phenylethanol, benzyl alcohol, chlorobutanol, chlorocresol, ethyl p-hydroxybenzoate or mixtures thereof.
80. The formulation according embodiment 79, wherein said preservative is present in a concentration from about 1mg/ml to 8mg/ml.
81.The formulation according embodiment 80, wherein said preservative is present in a concentration from about 2mg/ml to 6mg/ml.
82. A method for preparing the pharmaceutical formulation according embodiment 66, comprising the following steps:
   (1) dissolving a preservative, xylitol and a buffering agent in water for injection to prepare a solution;
   (2) dissolving the GLP-1 analogous in the above solution, adjusting the pH to the desired pH;
   (3) adding a stabilizer to the above solution to obtain the pharmaceutical formulation comprising GLP-1 analog in a concentration from 0.1mg/ml to 25mg/ml, a buffer with pH from 7.5 to 9.0, a stabilizer in a concentration from 0.001% to 0.5% (m/v), xylitol in a concentration from 0.5% to 10%(m/v), and preservative in a concentration from 0.1mg/ml to 10 mg/ml.

## Claims

1. A pharmaceutical formulation comprising a GLP-1 analog in a concentration from 0.1mg/ml to 25mg/ml, a buffer with pH from 7.5 to 9.0, polysorbate 80 at a concentration from 0.001% to 0.5% (m/v), poloxamer 188 at a concentration from 0.001% to 0.5% (m/v), xylitol at a concentration from 0.5% to 10% (m/v), and a preservative in a concentration from 0.1mg/ml to 10 mg/ml;
wherein the GLP-1 analog is selected from the group consisting of GLP-1 or its mutant, GLP-1(7-36)-amide or its mutant, GLP-1 (7-37) or its mutant, and GLP-1 derivatives which is chemically modified by introducing an organic substituent e.g. ester, alkyl or lipophilic group on one or more amino acid residues of the above peptide or its mutant thereof;
and wherein the term "mutant" designates the peptide GLP-1, GLP-1 (7-36)-amide or GLP-1 (7-37) wherein one or more amino acid residues of the peptide have been substituted by another amino acid residue and/or wherein one or more amino acid residues of the peptide have been deleted and/or wherein one or more amino acid residues have been added to the peptide, and wherein the mutant is a peptide GLP-1, GLP-1 (7-36) - amide or GLP-1 (7-37) wherein 6 or less amino acids have been substituted and/or added and/or deleted.

2. The formulation according claim 1, wherein the GLP-1 analog has a lipophilic substituent attached to the GLP-1 analog has 4-40 carbon atoms, 8-30 carbon atoms, 8-25 carbon atoms, 12-25 carbon atoms, or 14-18 carbon atoms.

3. The formulation according claim 1, wherein the GLP-1 analog is Arg³⁴Lys²⁶(N^{ε}-(γ-Glu(N^{α}-hexadecanoyl)))-GLP-1(7-37).

4. The formulation according claim 1, wherein the GLP-1 analog is selected from the group consisting of Gly⁸-GLP-1(7-36)-amide, Gly⁸-GLP-1(7-37), Val⁸-GLP-1(7-36)-amide, Val⁸-GLP-1(7-37), Val⁸Asp²²-GLP-1(7-37), Val⁸Asp²²-GLP-1(7-36)-amide, Val⁸Glu²²-GLP-1 (7-36)-amide, Val⁸Glu²²-GLP-1(7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1 (7-37), Val⁸His²²-GLP-1(7-36)-amide, Val⁸His²²-GLP-1(7-37); Arg²⁶-GLP-1(7-37); Arg³⁴-GLP-1(7-37); Lys³⁶-GLP-1(7-37); Arg^{26, 34}Lys³⁶-GLP-1(7-37); Arg^{26, 34}-GLP-1(7-37); Arg^{26, 34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly⁸His²²-GLP-1(7-37); Val⁸His²²-GLP-1(7-37); Met⁸His²²-GLP-1 (7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Val⁸Asp²²-GLP-1(7-37); Met⁸Asp²²-GLP-1(7-37); Gly⁸Glu²²-GLP-1(7-37); Val⁸Glu²²-GLP-1(7-37); Met8Glu²²-GLP-1(7-37); Gly⁸Lys²²-GLP-1(7-37); Val⁸Lys²²-GLP-1(7-37); Met⁸Lys²²-GLP-1(7-37); Gly⁸Arg²²-GLP-1(7-37); Val⁸Lys²²His³⁷-GLP-1 (7-37); Gly⁸Glu²²His³⁷-GLP-1(7-37); Val⁸Glu²²His³⁷-GLP-1(7-37); Met⁸Glu²²His³⁷-GLP-1(7-37); Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1 (7-37); Met⁸Arg²²His³⁷-GLP-1(7-37); Gly⁸His²²His³⁷-GLP-1(7-37); Val⁸His²²His³⁷-GLP-1(7-37); Met⁸His³³His³⁷-GLP-1(7-37); Gly⁸His³⁷-GLP-1(7-37); Val⁸His³⁷-GLP-1(7-37); Met⁸His³⁷-GLP-1(7-37); Gly⁸Asp²²His³⁷-GLP-1(7-37); Val⁸Asp²²His³⁷-GLP-1(7-37); Met⁸Asp²²His³⁷-GLP-1(7-37); Arg²⁶-GLP-1(7-36)-amide; Arg³⁴-GLP-1(7-36)-amide; Lys³⁶-GLP-1(7-36)-amide; Arg^{26, 34}Lys³⁶-GLP-1(7-36)-amide; Ar^{26, 34}-GLP-1(7-36)-amide; Arg^{26, 34}Lys⁴⁰-GLP-1(7-36)-amide; Arg²⁶Lys³⁶-GLP-1(7-36)-amide; Arg³⁴Lys³⁶-GLP-1(7-36)-amide; Gly⁸-GLP-1(7-36)-amide; Val⁸-GLP-1(7-36)-amide; Met⁸-GLP-1(7-36)-amide; Gly⁸Asp²²-GLP-1(7-36)-amide; Gly⁸Glu²²His³⁷-GLP-1(7-36)-amide; Val⁸Asp²²-GLP-1(7-36)-amide; Met⁸Asp²²-GLP-1(7-36)-amide; Gly⁸Glu²²-GLP-1(7-36)-amide; Val⁸Glu²²-GLP-1(7-36)-amide; Met⁸Glu²²-GLP-1(7-36)-amide; Gly⁸Lys²²-GLP-1(7-36)-amide; Val⁸Lys²²-GLP-1(7-36)-amide; Met⁸Lys²²-GLP-1(7-36)-amide; Gly⁸His²²His³⁷-GLP-1(7-36)-amide; Gly⁸Arg²²-GLP-1(7-3 6)-amide ; Val⁸Arg²²-GLP-1(7-3 6)-amide ; Met⁸Arg²²-GLP-1 (7-36)-amide; Gly⁸His²²-GLP-1(7-3 6)-amide ; Val⁸His²²-GLP-1(7-3 6)-amide ; Met⁸His²²-GLP-1(7-36)-amide; His³⁷-GLP-1(7-36)-amide; Val⁸Arg²²His³⁷-GLP-1(7-36)-amide; Met⁸Arg²²His³⁷-GLP-1(7-36)-amide; Gly⁸His³⁷-GLP-1(7-36)-amide; Val⁸His³⁷-GLP-1(7-36)-amide; Met⁸His³⁷-GLP-1(7-36)-amide; Gly⁸Asp²²His³⁷-GLP-1(7-36)-amide ; Val⁸Asp²²His³⁷-GLP-1(7-36)-amide; Met⁸Asp²²His³⁷-GLP-1(7-36)-amide; Val⁸Glu²²His³⁷-GLP-1(7-36)-amide; Met⁸Glu²²His³⁷-GLP-1(7-36)-amide; Gly⁸Lys²²His³⁷-GLP-1(7-36)-amide; Val⁸Lys²²His³⁷-GLP-1(7-36)-amide; Met⁸Lys²²His³⁷-GLP-1(7-36)-amide; Gly⁸Arg²²His³⁷-GLP-1(7-36)-amide; Val⁸His²²His³⁷-GLP-1(7-36)-amide; Met⁸His²²His³⁷-GLP-1(7-36)-amide; Ual⁸Trp¹⁹Glu²²-GLP-1(7-37), Val⁸Glu²²Val²⁵-GLP-1(7-37), Ual^{g}Tyr¹⁶Glu²²-GLP-1(7-37), Val⁸Trp¹⁶Glu²²-GLP-1(7-37), Val⁸Leu¹⁶Glu²²-GLP-1 (7-37), Val⁸Tyr¹⁸Glu²²-GLP-1(7-37), Val⁸Glu²²His³⁷GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), analogs thereof and derivatives of any of these.

5. The formulation according claim 1, wherein the concentration of the GLP-1 analog is from about 1mg/ml to 15mg/ml, more preferably from 3mg/ml to 10mg/ml, and most preferably 6mg/ml.

6. The formulation according claim 1, wherein said buffer is any buffer capable of maintaining the pH of the formulation in the aqueous solution at a pH from 7.5 to 9.0, which can be selected from the group consisting of phosphate buffer, a disodium hydrogen phosphate-Citrate buffer, TRIS, glycyl-glycine, N-bis (hydroxyethyl) glycine, sodium dihydrogen phosphate buffer, disodium hydrogen phosphate buffer, sodium acetate buffer, sodium carbonate buffer, sodium phosphate buffer, lysine buffer, arginine buffer or a mixture thereof.

7. The formulation according claim 6, wherein the pH of said buffer is from 7.5 to 8.5, more preferably from 8.0 to 8.5.

8. The formulation according claim 6, wherein the concentration of said buffer is from 5mg/ml to 100mg/ml, and more preferably from 10mg/ml to 30mg/ml.

9. The formulation according claim 1, wherein said xylitol is present in a concentration from 1% to 5% (m/v).

10. The formulation according claim 1, wherein said preservative is selected from the group consisting of phenol, o-cresol, m-cresol, p-cresol, methyl p-hydroxybenzoate, propyl p-hydroxybenzoate, 2-phenoxyethanol, p-hydroxybenzene butyl formate, 2-phenylethanol, benzyl alcohol, chlorobutanol, chlorocresol, ethyl p-hydroxybenzoate or mixtures thereof.

11. The formulation according to claim 1,wherein the formulation comprises liraglutide at a concentration from 0.1mg/ml to 25mg/ml , pH from 7.5 to 9.0, disodium hydrogen phosphate buffer at a concentration from 5 mmol/L to 100 mmol/L, polysorbate 80 or poloxamer 188 at a concentration from 0.001% to 0.5% (m/v), xylitol at a concentration from 0.5% to 10% (m/v), phenol or m-cresol at a concentration from 0.1 mg/ml to 10 mg/ml.

12. The formulation according to claim 1, wherein the formulation comprises liraglutide at a concentration from 0.1 mg/ml to 25 mg/ml, pH from 7.5 to 9.0, disodium hydrogen phosphate buffer at a concentration from 5 mmol/L to 100 mmol/L, polysorbate 80 at a concentration from 0.001% to 0.5% (m/v), poloxamer 188 at a concentration from 0.001% to 0.5% (m/v), xylitol at a concentration from 0.5% to 10% (m/v), phenol or m-cresol at a concentration from 0.1 mg/ml to 10 mg/ml.

13. A method for preparing the pharmaceutical formulation according claim 1, comprising the following steps:
(1) dissolving a preservative, xylitol and a buffering agent in water for injection to prepare a solution;
(2) dissolving the GLP-1 analog defined in claim 1 in the above solution, adjusting the pH to the desired pH;
(3) adding a stabilizer to the above solution to obtain the pharmaceutical formulation comprising the GLP-1 analog in a concentration from 0.1 mg/ml to 25 mg/ml, a buffer with pH from 7.5 to 9.0, a stabilizer in a concentration from 0.001% to 0.5% (m/v), xylitol in a concentration from 0.5% to 10%(m/v), and a preservative in a concentration from 0.1 mg/ml to 10 mg/ml.

## Patentansprüche

1. Pharmazeutische Formulierung umfassend ein GLP-1-Analogon in einer Konzentration von 0,1mg/ml bis 25mg/ml, einen Puffer mit pH von 7,5 bis 9,0, Polysorbat 80 in einer Konzentration von 0,001% bis 0,5% (m/v), Poloxamer 188 in einer Konzentration von 0,001% bis 0,5% (m/v), Xylitol in einer Konzentration von 0,5% bis 10% (m/v), und ein Konservierungsmittel in einer Konzentration von 0,1mg/ml bis 10 mg/ml;
wobei das GLP-1 Analogon ausgewählt ist aus der Gruppe bestehend aus GLP-1 oder seiner Mutante, GLP-1(7-36)-Amid oder seiner Mutante, GLP-1 (7-37) oder seiner Mutante, und GLP-1 Derivaten, die chemisch modifiziert sind, indem ein organischer Substituent z.B. Ester, Alkyl oder lipophile Gruppe an einem oder mehreren Aminosäurerest(en) des oben genannten Peptids oder seiner Mutante davon eingeführt wird;
und wobei der Begriff "Mutant" das Peptid GLP-1, GLP-1 (7-36)-Amid oder GLP-1 (7-37) bezeichnet, wobei ein oder mehrere Aminosäurerest(e) des Peptids durch einen anderen Aminosäurerest ersetzt wurde(n) und/oder wobei ein oder mehrere Aminosäurerest(e) des Peptids deletiert wurde(n) und/oder wobei dem Peptid ein oder mehrere Aminosäurerest(e) hinzugefügt wurde(n), und wobei der Mutant ein Peptid GLP-1, GLP-1 (7-36)-Amid oder GLP-1 (7-37) ist, wobei 6 oder weniger Aminosäuren ersetzt und/oder hinzugefügt und/oder deletiert wurden.

2. Formulierung nach Anspruch 1, wobei das GLP-1-Analogon einen lipophilen Substituenten gebunden an das GLP-1-Analogon hat, hat 4-40 Kohlenstoffatome, 8-30 Kohlenstoffatome, 8-25 Kohlenstoffatome, 12-25 Kohlenstoffatome, oder 14-18 Kohlenstoffatome.

3. Formulierung nach Anspruch 1, wobei das GLP-1 Analogon Arg³⁴,Lys²⁶(N^{ε}-(γ-Glu(N^{α}-hexadecanoyl))-GLP-1(7-37) ist.

4. Formulierung nach Anspruch 1, wobei das GLP-1 Analogon ausgewählt ist aus der Gruppe bestehend aus Gly⁸-GLP-1(7-36)-Amid, Gly⁸-GLP-1(7-37), Val⁸-GLP-1(7-36)-Amid, Val⁸-GLP-1(7-37), Val⁸Asp²²-GLP-1(7-37), Val⁸Asp²²-GLP-1(7-36)-Amid, Val⁸Glu²²-GLP-1 (7-36)-Amid, Val⁸Glu²²-GLP-1(7-37), Val⁸Lys²²-GLP-1(7-36)-Amid, Val⁸Lys²²-GLP-1(7-37), Val⁸Arg²²-GLP-1(7-36)-Amid, Val⁸Arg²²-GLP-1(7-37), Val⁸His²²-GLP-1(7-36)-Amid, Val⁸His²²-GLP-1(7-37); Arg²⁶-GLP-1(7-37); Arg³⁴-GLP-1(7-37); Lys³⁶-GLP-1(7-37); Arg^{26, 34}Lys³⁶-GLP-1(7-37); Arg^{26, 34}-GLP-1(7-37); Arg^{26, 34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly⁸His²²-GLP-1(7-37); Val⁸His²²-GLP-1(7-37); Met⁸His²²-GLP-1(7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Ual⁸Asp²²-GLP-1(7-37); Met⁸Asp²²-GLP-1(7-37); Gly⁸Glu²²-GLP-1(7-37); Val⁸Glu²²-GLP-1(7-37); Met8Glu²²-GLP-1(7-37); Gly⁸Lys²²-GLP-1(7-37); Val⁸Lys²²-GLP-1(7-37); Met⁸Lys²²-GLP-1(7-37); Gly1(7-37); Val⁸Lys²²His³⁷-GLP-1(7-37); Gly⁸Glu²²His³⁷-GLP-1(7-37); Val⁸Glu²²His³⁷-GLP-1(7-37); Met⁸Glu²²His³⁷-GLP-1(7-37); Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1(7-37); Met⁸Arg²²His³⁷-GLP-1(7-37); Gly⁸His²²His³⁷-GLP-1(7-37); Val⁸His²²His³⁷-GLP-1(7-37); Met⁸His³³His³⁷-GLP-1(7-37); Gly⁸His³⁷-GLP-1(7-37); Val⁸His³⁷-GLP-1(7-37); Met⁸His³⁷-GLP-1(7-37); Gly⁸Asp²²His³⁷-GLP-1(7-37); Val⁸Asp²²His³⁷-GLP-1(7-37); Met⁸Asp²²His³⁷-GLP-1(7-37); Arg²⁶-GLP-1(7-36)-Amid; Arg³⁴-GLP-1(7-36)-Amid; Lys³⁶-GLP-1(7-36)-Amid; Arg^{26, 34}Lys³⁶-GLP-1(7-36)-Amid; Ar^{26, 34}-GLP-1(7-36)-Amid; Arg^{26, 34}Lys⁴⁰-GLP-1(7-36)-Amid; Arg²⁶Lys³⁶-GLP-1(7-36)-Amid; Arg³⁴Lys³⁶-GLP-1(7-36)-Amid; Gly⁸-GLP-1(7-36)-Amid; Val⁸-GLP-1(7-36)-Amid; Met⁸-GLP-1(7-36)-Amid; Gly⁸Asp²²-GLP-1(7-36)-Amid; Gly⁸Glu²²His³⁷-GLP-1(7-36)-Amid; Val⁸Asp²²-GLP-1(7-36)-Amid; Met⁸Asp²²-GLP-1(7-36)-Amid; Gly⁸Glu²²-GLP-1(7-36)-Amid; Val⁸Glu²²-GLP-1(7-36)-Amid; Met⁸Glu²²-GLP-1(7-36)-Amid; Gly⁸Lys²²-GLP-1(7-36)-Amid; Val⁸Lys²²-GLP-1(7-36)-Amid; Met⁸Lys²²-GLP-1(7-36)-Amid; Gly⁸His²²His³⁷-GLP-1(7-36)-Amid; Gly⁸Arg²²-GLP-1(7-36)-Amid; Val⁸Arg²²-GLP-1(7-36)-Amid; Met⁸Arg²²-GLP-1(7-36)-Amid; Gly⁸His²²-GLP-1(7-36)-Amid; Val⁸His²²-GLP-1(7-36)-Amid; Met⁸His²²-GLP-1(7-36)-Amid; His³⁷-GLP-1(7-36)-Amid; Val⁸Arg²²His³⁷-GLP-1(7-36)-Amid; Met⁸Arg²²His³⁷-GLP-1(7-36)-Amid; Gly⁸His³⁷-GLP-1(7-36)-Amid; Val⁸His³⁷-GLP-1(7-36)-Amid; Met⁸His³⁷-GLP-1(7-36)-Amid; Gly⁸Asp²²His³⁷-GLP-1(7-36)-Amid; Val⁸Asp²²His³⁷-GLP-1(7-36)-Amid; Met⁸Asp²²His³⁷-GLP-1(7-36)-Amid; Val⁸Glu²²His³⁷-GLP-1(7-36)-Amid; Met⁸Glu²²His³⁷-GLP-1(7-36)-Amid; Gly⁸Lys²²His³⁷-GLP-1(7-36)-Amid; Val⁸Lys²²His³⁷-GLP-1(7-36)-Amid; Met⁸Lys²²His³⁷-GLP-1(7-36)-Amid; Gly⁸Arg²²His³⁷-GLP-1(7-36)-Amid; Val⁸His²²His³⁷-GLP-1(7-36)-Amid; Met⁸His²²His³⁷-GLP-1(7-36)-Amid; Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1(7-37), Val⁸Tyr¹⁸Glu²²-GLP-1(7-37), Val⁸Glu²²His³⁷GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), Analoga davon und Derivate von irgendeinem von diesen.

5. Formulierung nach Anspruch 1, wobei die Konzentration des GLP-1-Analogons von etwa 1mg/ml bis 15mg/ml, bevorzugter von 3mg/ml bis 10mg/ml und am bevorzugtesten 6mg/ml ist.

6. Formulierung nach Anspruch 1, wobei der Puffer irgendein Puffer ist, der in der Lage ist, den pH der Formulierung in der wässrigen Lösung bei einem pH von 7,5 bis 9,0 aufrechtzuerhalten, der ausgewählt werden kann aus der Gruppe bestehend aus Phosphatpuffer, einem Dinatriumhydrogenphosphat-Citratpuffer, TRIS, Glycylglycin, N-bis (hydroxyethyl)-Glyin, Natriumdihydrogenphosphatpuffer, Dinatriumhydrogenphosphatpuffer, Natriumacetatpuffer, Natriumkarbonatpuffer, Natriumphosphatpuffer, Lysinpuffer, Argininpuffer oder einer Mischung davon.

7. Formulierung nach Anspruch 6, wobei der pH des Puffers von 7,5 bis 8,5, bevorzugter von 8,0 bis 8,5 ist.

8. Formulierung nach Anspruch 6, wobei die Konzentration des Puffers von 5mg/ml bis 100mg/ml, und bevorzugter von 10mg/ml bis 30mg/ml ist.

9. Formulierung nach Anspruch 1, wobei das Xylitol in einer Konzentration von 1 % bis 5 % (m/v) vorhanden ist.

10. Formulierung nach Anspruch 1, wobei das Konservierungsmittel ausgewählt ist aus der Gruppe bestehend aus Phenol, o-Cresol, m-Cresol, p-Cresol, Methyl p-Hydroxybenzoat, Propyl p-Hydroxybenzoat, 2-Phenoxyethanol, p-Hydroxybenzol-Butylformat, 2-Phenylethanol, Benzylalkohol, Chlorobutanol, Chlorocresol, Ethyl p-Hydroxybenzoat oder Mischungen davon.

11. Formulierung nach Anspruch 1, wobei die Formulierung Liraglutid in einer Konzentration von 0,1mg/ml bis 25mg/ml, pH von 7,5 bis 9,0, Dinatriumhydrogenphosphatpuffer in einer Konzentration von 5 mmol/L bis 100 mmol/L, Polysorbat 80 oder Poloxamer 188 in einer Konzentration von 0,001% bis 0,5% (m/v), Xylitol in einer Konzentration von 0,5% bis 10% (m/v), Phenol oder m-Cresol in einer Konzentration von 0,1 mg/ml bis 10 mg/ml umfasst.

12. Formulierung nach Anspruch 1, wobei die Formulierung Liraglutid in einer Konzentration von 0,1 mg/ml bis 25 mg/ml, pH von 7,5 bis 9,0, Dinatriumhydrogenphosphatpuffer in einer Konzentration von 5 mmol/L bis 100 mmol/L, Polysorbat 80 in einer Konzentration von 0,001% bis 0,5% (m/v), Poloxamer 188 in einer Konzentration von 0,001% bis 0,5% (m/v), Xylitol in einer Konzentration von 0,5% bis 10% (m/v), Phenol oder m-Cresol in einer Konzentration von 0,1 mg/ml bis 10 mg/ml umfasst.

13. Verfahren zum Herstellen der pharmazeutischen Formulierung nach Anspruch 1, die folgenden Schritte umfassend:
(1) Lösen eines Konservierungsmittels, Xylitols und eines Puffermittels in Wasser zur Injektion zum Herstellen einer Lösung;
(2) Auflösen des GLP-1-Analogons definiert in Anspruch 1 in der oben genannten Lösung, Einstellen des pHs auf den gewünschten pH;
(3) Zugeben eines Stabilisators zu der oben genannten Lösung, um die pharmazeutische Formulierung zu erhalten umfassend das GLP-1-Analogon in einer Konzentration von 0,1 mg/ml bis 25 mg/ml, einen Puffer mit pH von 7,5 bis 9,0, einen Stabilisator in einer Konzentration von 0,001% bis 0,5% (m/v), Xylitol in einer Konzentration von 0,5% bis 10% (m/v), und ein Konservierungsmittel in einer Konzentration von 0,1mg/ml bis 10 mg/ml.

## Revendications

1. Un formulation pharmaceutique comprenant un analogue du GLP-1 à une concentration allant de 0,1mg/ml à 25mg/ml, un tampon avec un pH allant de 7,5 à 9,0, du polysorbate 80 à une concentration allant de 0,001% à 0,5% (m/v), du poloxamer 188 à une concentration allant de 0,001% à 0,5% (m/v), du xylitol à une concentration allant de 0,5% à 10% (m/v) et un conservateur à une concentration allant de 0,1mg/ml à 10mg/ml ;
l'analogue du GLP-1 étant choisi dans le groupe consistant en GLP-1 ou son mutant, GLP-1(7-36)-amide ou son mutant, GLP-1(7-37) ou son mutant, et des dérivés du GLP-1 modifiés chimiquement par introduction d'un substituant organique, p. un groupe ester, alkyle ou lipophile sur un ou plusieurs résidus d'acides aminés du peptide ci-dessus ou du mutant de celui-ci ; et
le terme "mutant" désignant le peptide GLP-1, GLP-1 (7-36)-amide ou GLP-1 (7-37) dans lequel un ou plusieurs résidus d'acides aminés du peptide ont été substitués par un autre résidu d'acide aminé et / ou dans lequel un ou plusieurs résidus d'acide aminé du peptide ont été supprimés et / ou dans lequel un ou plusieurs résidus d'acide aminé ont été ajoutés au peptide, et le mutant étant un peptide GLP-1, GLP-1 (7-36) -amide ou GLP-1 (7-37) dans lequel 6 acides aminés ou moins ont été substitués et / ou ajoutés et / ou supprimés.

2. La formulation selon la revendication 1, dans laquelle l'analogue GLP-1 a un substituant lipophile fixé à l'analogue GLP-1 a 4-40 atomes de carbone, 8-30 atomes de carbone, 8-25 atomes de carbone, 12-25 atomes de carbone, ou 14-18 atomes de carbone.

3. La formulation selon la revendication 1, dans laquelle l'analogue du GLP-1 est Arg³⁴,Lys²⁶(N^{ε}-(γ-Glu(N^{α}-hexadécanoyl)))-GLP-1(7-37).

4. La formulation selon la revendication 1, dans laquelle l'analogue du GLP-1 est choisi dans le groupe consistant en Gly⁸-GLP-1(7-36)-amide, Gly⁸-GLP-1(7-37), Val⁸-GLP-1(7-36)-amide, Val⁸-GLP-1(7-37), Val⁸Asp²²-GLP-1(7-37), Val⁸Asp²²-GLP-1(7-36)-amide, Val⁸Glu²²-GLP-1 (7-36)-amide, Val⁸Glu²²-GLP-1(7-37), Val⁸Lys²²-GLP-1(7-36)-amide, Val⁸Lys²²-GLP-1(7-37), Val⁸Arg²²-GLP-1(7-36)-amide, Val⁸Arg²²-GLP-1 (7-37), Val⁸His²²-GLP-1(7-36)-amide, Val⁸His²²-GLP-1(7-37); Arg²⁶-GLP-1(7-37); Arg³⁴-GLP-1(7-37); Lys³⁶-GLP-1(7-37) ; Arg^{26, 34}Lys³⁶-GLP-1(7-37); Arg^{26, 34}-GLP-1(7-37); Arg^{26, 34}Lys⁴⁰-GLP-1(7-37); Arg²⁶Lys³⁶-GLP-1(7-37); Arg³⁴Lys³⁶-GLP-1(7-37); Val⁸Arg²²-GLP-1(7-37); Met⁸Arg²²-GLP-1(7-37); Gly⁸His²²-GLP-1(7-37); Val⁸His²²-GLP-1(7-37); Met⁸His²²-GLP-1(7-37); His³⁷-GLP-1(7-37); Gly⁸-GLP-1(7-37); Val⁸-GLP-1(7-37); Met⁸-GLP-1(7-37); Gly⁸Asp²²-GLP-1(7-37); Val⁸Asp²²-GLP-1(7-37); Met⁸Asp²²-GLP-1(7-37); Gly⁸Glu²²-GLP-1(7-37); Val⁸Glu²²-GLP-1(7-37); Met⁸Glu²²-GLP-1(7-37); Gly⁸Lys²²-GLP-1(7-37); Val⁸Lys²²-GLP-1(7-37) ; Met⁸Lys²²-GLP-1(7-37); Gly⁸Arg²²-GLP-1(7-37); Val⁸Lys²²His³⁷-GLP-1 (7-37) ; Gly⁸Glu²²His³⁷-GLP-1(7-37); Val⁸Glu²²His³⁷-GLP-1(7-37); Met⁸Glu²²His³⁷-GLP-1(7-37) ; Gly⁸Lys²²His³⁷-GLP-1(7-37); Met⁸Lys²²His³⁷-GLP-1(7-37); Gly⁸Arg²²His³⁷-GLP-1(7-37); Val⁸Arg²²His³⁷-GLP-1(7-37) ; Met⁸Arg²²His³⁷-GLP-1(7-37) ; Gly⁸His²²His³⁷-GLP-1(7-37); Val⁸His²²His³⁷-GLP-1(7-37); Met⁸His³³His³⁷-GLP-1(7-37); Gly⁸His³⁷-GLP-1(7-37); Val⁸His³⁷-GLP-1(7-37); Met⁸His³⁷-GLP-1(7-37); Gly⁸Asp²²His³⁷-GLP-1(7-37); Val⁸Asp²²His³⁷-GLP-1(7-37); Met⁸Asp²²His³⁷-GLP-1(7-37); Arg²⁶-GLP-1(7-36)-amide; Arg³⁴-GLP-1(7-36)-amide; Lys³⁶-GLP-1(7-36)-amide; Arg^{26, 34}Lys³⁶-GLP-1(7-36)-amide; Ar^{26, 34}-GLP-1(7-36)-amide; Arg^{26, 34}Lys⁴⁰-GLP-1(7-36)-amide; Arg²⁶Lys³⁶-GLP-1(7-36)-amide; Arg³⁴Lys³⁶-GLP-1(7-36)-amide; Gly⁸-GLP-1(7-36)-amide; Val⁸-GLP-1(7-36)-amide; Met⁸-GLP-1(7-36)-amide; Gly⁸Asp²²-GLP-1(7-36)-amide; Gly⁸Glu²²His³⁷-GLP-1(7-36)-amide; Val⁸Asp²²-GLP-1(7-36)-amide; Met⁸Asp²²-GLP-1(7-36)-amide; Gly⁸Glu²²-GLP-1(7-36)-amide; Val⁸Glu²²-GLP-1(7-36)-amide; Met⁸Glu²²-GLP-1(7-36)-amide; Gly⁸Lys²²-GLP-1(7-36)-amide ; Val⁸Lys²²-GLP-1(7-36)-amide; Met⁸Lys²²-GLP-1(7-36)-amide; Gly⁸His²²His³⁷-GLP-1(7-36)-amide; Gly⁸Arg²²-GLP-1(7-36)-amide; Val⁸Arg²²-GLP-1(7-36)-amide; Met⁸Arg²²-GLP-1(7-36)-amide; Gly⁸His²²-GLP-1(7-36)-amide; Val⁸His²²-GLP-1(7-36)-amide; Met⁸His²²-GLP-1(7-36)-amide; His³⁷-GLP-1(7-36)-amide ; Val⁸Arg²²His³⁷-GLP-1(7-36)-amide; Met⁸Arg²²His³⁷-GLP-1(7-36)-amide; Gly⁸His³⁷-GLP-1(7-36)-amide; Val⁸His³⁷-GLP-1(7-36)-amide; Met⁸His³⁷-GLP-1(7-36)-amide ; Gly⁸Asp²²His³⁷-GLP-1(7-36)-amide; Val⁸Asp²²His³⁷-GLP-1(7-36)-amide; Met⁸Asp²²His³⁷-GLP-1(7-36)-amide; Val⁸Glu²²His³⁷-GLP-1(7-36)-amide; Met⁸Glu²²His³⁷-GLP-1(7-36)-amide; Gly⁸Lys²²His³⁷-GLP-1(7-36)-amide; Val⁸Lys²²His³⁷-GLP-1(7-36)-amide; Met⁸Lys²²His³⁷-GLP-1(7-36)-amide; Gly⁸Arg²²His³⁷-GLP-1(7-36)-amide, Val⁸His²²His³⁷-GLP-1(7-36)-amide; Met⁸His²²His³⁷-GLP-1(7-36)-amide; Val⁸Trp¹⁹Glu²²-GLP-1(7-37), Val⁸Glu²²Val²⁵-GLP-1(7-37), Val⁸Tyr¹⁶Glu²²-GLP-1(7-37), Val⁸Trp¹⁶Glu²²-GLP-1(7-37), Val⁸Leu¹⁶Glu²²-GLP-1 (7-37), Val⁸Tyr¹⁸Glu²²-GLP-1(7-37), Val⁸Glu²²His³⁷GLP-1(7-37), Val⁸Glu²²Ile³³-GLP-1(7-37), Val⁸Trp¹⁶Glu²²Val²⁵Ile³³-GLP-1(7-37), Val⁸Trp¹⁶Glu²²Ile³³-GLP-1(7-37), Val⁸Glu²²Val²⁵Ile³³-GLP-1(7-37), Val⁸Trp¹⁶Glu²²Val²⁵-GLP-1(7-37), les analogues de ceux-ci et dérivés de l'un quelconque de ceux-ci.

5. La formulation selon la revendication 1, dans laquelle la concentration de l'analogue du GLP-1 est d'environ 1mg/ml à 15mg/ml, de façon préférée de 3mg/ml à 10mg/ml, et de manière la plus préférée, de 6mg/ml.

6. La formulation selon la revendication 1, dans laquelle ledit tampon est tout tampon apte à maintenir le pH de la formulation dans la solution aqueuse à un pH allant de 7,5 à 9,0, qui peut être choisi dans le groupe constitué par un tampon phosphate, un tampon hydrogéno-phosphate-citrate disodique, TRIS, de la glycyl-glycine, de la glycine N-bis (hydroxyéthyl), un tampon phosphate dihydrogène sodium, un tampon hydrogénophosphate disodique, un tampon acétate de sodium, un tampon carbonate de sodium, un tampon phosphate de sodium, un tampon lysine, un tampon arginine ou un mélange de ceux-ci.

7. La formulation selon la revendication 6, dans laquelle le pH dudit tampon est de 7,5 à 8,5, de façon encore préférée de 8,0 à 8,5.

8. La formulation selon la revendication 6, dans laquelle la concentration dudit tampon est de 5mg/ml à 100mg/ml, et de façon encore préférée de 10mg/ml à 30mg/ml.

9. La formulation selon la revendication 1, dans laquelle ledit xylitol est présent à une concentration allant de 1% à 5% (m/v).

10. La formulation selon la revendication 1, dans laquelle ledit conservateur est choisi dans le groupe consistant en le phénol, l'o-crésol, le m-crésol, le p-crésol, le p-hydroxybenzoate de méthyle, le p-hydroxybenzoate de propyle, le 2-phénoxyéthanol, formiate de p-hydroxybenzène butyle, le 2-phényléthanol, l'alcool benzylique, le chlorobutanol, le chlorocrésol, le p-hydroxybenzoate d'éthyle ou les mélanges de ceux-ci.

11. La formulation selon la revendication 1, dans laquelle la formulation comprend du liraglutide à une concentration allant de 0, 1mg/ml à 25mg/ml, un pH allant de 7,5 à 9,0, un tampon d'hydrogénophosphate disodique à une concentration allant de 5 mmol/L à 100 mmol/L, du polysorbate 80 ou du poloxamer 188 à une concentration allant de 0,001% à 0,5% (m/v), du xylitol à une concentration allant de 0,5% à 10% (m/v), du phénol ou du m-crésol à une concentration allant de 0,1mg/ml à 10mg/ml.

12. La formulation selon la revendication 1, dans laquelle la formulation comprend du liraglutide à une concentration allant de 0, 1mg/ml à 25mg/ml, un pH allant de 7,5 à 9,0, un tampon d'hydrogénophosphate disodique à une concentration allant de 5 mmol/L à 100 mmol/L, du polysorbate 80 à une concentration allant de 0,001% à 0,5% (m/v), du poloxamer 188 à une concentration allant de 0,001% à 0,5% (m/v), du xylitol à une concentration allant de 0,5% à 10% (m/v), du phénol ou du m-crésol à une concentration allant de 0,1 mg/ml à 10 mg/ml.

13. Un procédé de préparation de la formulation pharmaceutique selon la revendication 1, comprenant les étapes suivantes :
(1) dissoudre un conservateur, du xylitol et un agent tampon dans de l'eau pour injection afin de préparer une solution ;
(2) dissoudre l'analogue du GLP-1 défini dans la revendication 1 dans la solution ci-dessus, ajuster le pH au pH souhaité ;
(3) ajouter un stabilisant à la solution ci-dessus pour obtenir la formulation pharmaceutique comprenant l'analogue du GLP-1 à une concentration allant de 0,1mg/ml à 25mg/ml, un tampon avec un pH allant de 7,5 à 9,0, un stabilisant à une concentration allant de 0,001% à 0,5% (m/v), du xylitol à une concentration allant de 0,5% à 10%(m/v) et un conservateur à une concentration allant de 0,1mg/ml à 10mg/ml.
